# EUROPEAN PATENT APPLICATION

(11) **EP 2 308 897 A1**
(43) Date of publication of application: **13.04.2011**
(21) Application number: 09305964.0
(22) Date of filing: 09.10.2009
(51) Int. Cl.: C07K 16/28, A61P 35/00

(54) **Chimeric antibodies specific for CD151 and use thereof in the treatment of cancer**

(71) Applicant: PIERRE FABRE MEDICAMENT, 92100 Boulogne-Billancourt (FR)
(72) Inventor: Haeuw, Jean-François, 74160, Beaumont (FR)
(74) Representative: Warcoin, Jacques

(57) **Abstract**

The present invention relates to new antibodies capable of binding specifically to the human CD151 protein, especially monoclonal antibodies of murine origin, which are chimeric and humanised, and also to the amino acid and nucleic sequences coding for those antibodies. The invention also includes use of those antibodies as medicaments for the prophylactic and/or therapeutic treatment of cancers and in diagnostic methods or kits for diseases associated with overexpression of the CD151 protein. Finally, the invention includes products and/or compositions comprising such antibodies in association with antibodies and/or anti-cancer agents or conjugated with toxins and/or radioelements and their use in the prevention and/or treatment of certain cancers.

SEQUENCE LISTING

<110>  Pierre Fabre Médicament

<120>  New chimeric antibodies and their uses for the treatment of
       cancer

<130>  D28060

<160>  36

<170>  PatentIn version 3.3

<210>  1
<211>  10
<212>  PRT
<213>  mus musculus

<400>  1

Ala Ser Val Glu Tyr Tyr Gly Thr Ser Leu
1               5                   10


<210>  2
<211>  3
<212>  PRT
<213>  mus musculus

<400>  2

Glu Ala Ser
1


<210>  3
<211>  9
<212>  PRT
<213>  mus musculus

<400>  3

Gln Gln Ser Arg Lys Ala Pro Tyr Thr
1               5


<210>  4
<211>  8
<212>  PRT
<213>  mus musculus

<400>  4

Gly Phe Thr Phe Ser Thr Tyr Thr
1               5


<210>  5
<211>  8
<212>  PRT
<213>  mus musculus

<400>  5

Ile Ser Ser Gly Gly Gly Thr Thr
1               5


<210>  6
<211>  11
<212>  PRT
<213>  mus musculus

<400>  6

Ala Thr Pro Arg Ile Gly Thr Gly Phe Ala Tyr
1               5                   10


<210>  7
<211>  218
<212>  PRT
<213>  Artificial sequence

<220>
<223>  Chimeric anti-CD151 antibody light chain derived from the
       murine 203B6 monoclonal antibody

<400>  7

Asp Ile Val Leu Ser Gln Ser Pro Ala Ser Leu Ala Leu Ser Leu Gly
1               5                   10                  15


Gln Arg Ala Thr Ile Ser Cys Arg Ala Ser Ala Ser Val Glu Tyr Tyr
            20                  25                  30


Gly Thr Ser Leu Met His Trp Tyr Gln Gln Lys Pro Gly Gln Pro Pro
        35                  40                  45


Lys Leu Leu Ile Tyr Glu Ala Ser Asn Val Glu Ser Gly Val Pro Ala
    50                  55                  60


Arg Phe Ser Gly Ser Gly Ser Gly Thr Asp Phe Ser Leu Asn Ile His
65                  70                  75                  80


Pro Val Glu Glu Asp Asp Leu Ala Ile Tyr Phe Cys Gln Gln Ser Arg
                85                  90                  95


Lys Ala Pro Tyr Thr Phe Gly Gly Gly Thr Lys Leu Glu Ile Lys Arg
            100                 105                 110


Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210>  8
<211>  448
<212>  PRT
<213>  Artificial sequence

<220>
<223>  Chimeric anti-CD151 antibody heavy chain derived from the
       murine 203B6 monoclonal antibody

<400>  8

Glu Val Lys Leu Val Glu Ser Gly Gly Gly Leu Val Gln Pro Gly Gly
1               5                   10                  15

Ser Leu Lys Leu Ser Cys Ala Ala Ser Gly Phe Thr Phe Ser Thr Tyr
            20                  25                  30

Thr Met Ser Trp Val Arg Gln Thr Pro Glu Lys Arg Leu Glu Trp Val
        35                  40                  45

Ala Tyr Ile Ser Ser Gly Gly Gly Thr Thr Tyr Tyr Pro Asp Thr Val
    50                  55                  60

Lys Gly Arg Phe Thr Ile Ser Arg Asp Asn Ala Arg Asn Thr Leu Tyr
65                  70                  75                  80

Leu Gln Met Asn Ser Leu Lys Ser Glu Asp Thr Ala Met Tyr Tyr Cys
                85                  90                  95

Ala Thr Pro Arg Ile Gly Thr Gly Phe Ala Tyr Trp Gly Gln Gly Thr
            100                 105                 110

Leu Val Thr Val Ser Ala Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Arg Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Glu Glu Met Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
        435                 440                 445

<210>  9
<211>  445
<212>  PRT
<213>  Artificial sequence

<220>
<223>  Chimeric anti-CD151 antibody heavy chain derived from the
       murine 203B6 monoclonal antibody

<400>  9

Glu Val Lys Leu Val Glu Ser Gly Gly Gly Leu Val Gln Pro Gly Gly
1               5                   10                  15

Ser Leu Lys Leu Ser Cys Ala Ala Ser Gly Phe Thr Phe Ser Thr Tyr
            20                  25                  30

Thr Met Ser Trp Val Arg Gln Thr Pro Glu Lys Arg Leu Glu Trp Val
        35                  40                  45

Ala Tyr Ile Ser Ser Gly Gly Gly Thr Thr Tyr Tyr Pro Asp Thr Val
    50                  55                  60

Lys Gly Arg Phe Thr Ile Ser Arg Asp Asn Ala Arg Asn Thr Leu Tyr
65                  70                  75                  80

Leu Gln Met Asn Ser Leu Lys Ser Glu Asp Thr Ala Met Tyr Tyr Cys
                85                  90                  95

Ala Thr Pro Arg Ile Gly Thr Gly Phe Ala Tyr Trp Gly Gln Gly Thr

            100                 105                 110

Leu Val Thr Val Ser Ala Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Ser Lys Tyr Gly Pro Pro Cys
    210                 215                 220

Pro Ser Cys Pro Ala Pro Glu Phe Leu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser Gln Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Gly Leu Pro Ser Ser Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Gln Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Arg Leu Thr Val Asp Lys Ser Arg Trp Gln
                405                 410                 415

Glu Gly Asn Val Phe Ser Cys Ser Val Met His Glu Ala Leu His Asn
            420                 425                 430

His Tyr Thr Gln Lys Ser Leu Ser Leu Ser Leu Gly Lys
        435                 440                 445

<210>  10
<211>  30
<212>  DNA
<213>  mus musculus

<400>  10
gccagtgttg aatattatgg cacaagttta                                        30

<210>  11
<211>  9
<212>  DNA
<213>  mus musculus

<400>  11
gaagcatcc                                                                9

<210>  12
<211>  27
<212>  DNA
<213>  mus musculus

<400>  12
cagcaaagta ggaaggctcc ttacacg                                           27

<210>  13
<211>  24
<212>  DNA
<213>  mus musculus

<400>  13
ggattcactt tcagtaccta tacc                                              24

<210>  14
<211>  24
<212>  DNA
<213>  mus musculus

<400>  14
attagtagtg gtggtggtac tacc                                              24


<210>  15
<211>  33
<212>  DNA
<213>  mus musculus

<400>  15
gcaactcccc gaattgggac ggggtttgct tac                                    33


<210>  16
<211>  654
<212>  DNA
<213>  Artificial sequence

<220>
<223>  Chimeric  anti-CD151 antibody light chain derived from the
       murine 203B6 monoclonal antibody

<400>  16
gacatcgtgc tgtcccagag ccccgcctct ctggccctga gcctgggaca gcgggccacc      60

atctcttgcc gggccagcgc cagcgtggag tactacggca ccagcctgat gcactggtat     120

cagcagaagc ccggccagcc ccccaagctg ctgatctacg aggccagcaa cgtggagagc     180

ggcgtgcccg ccagattcag cggcagcggc tccggcaccg acttcagcct gaacatccac     240

cccgtggaag aggacgacct ggccatctac ttttgccagc agagccggaa ggccccctac     300

acctttggcg gcggaacaaa gctggaaatc aagcgtacgg tggccgctcc cagcgtgttc     360

atcttccccc caagcgacga gcagctgaag agcggcaccg ccagcgtggt gtgtctgctg     420

aacaacttct accccaggga ggccaaggtg cagtggaagg tggacaacgc cctgcagagc     480

ggcaacagcc aggagagcgt caccgagcag gacagcaagg actccaccta cagcctgagc     540

agcaccctga ccctgagcaa ggccgactac gagaagcaca aggtgtacgc ctgtgaggtg     600

acccaccagg gcctgtccag ccccgtgacc aagagcttca acaggggcga gtgc           654


<210>  17
<211>  1344
<212>  DNA
<213>  Artificial sequence

<220>
<223>  Chimeric anti-CD151 antibody heavy chain derived from the
       murine 203B6 monoclonal antibody

<400>  17
gaagtgaaac tggtggagtc tggcggcgga ctggtgcagc caggcggcag cctgaagctg      60

tcctgcgccg ccagcggctt caccttcagc acctacacca tgagctgggt gcgccagacc     120

cccgagaagc ggctggaatg ggtggcctac atcagcagcg gcggagggac cacctactac     180

cccgacaccg tgaagggccg gttcaccatc agccgggaca acgcccggaa caccctgtac     240

ctgcagatga acagcctgaa gtccgaggac accgccatgt actactgcgc caccccccgg     300

atcggcaccg gcttcgccta ctggggccag ggcaccctgg tgaccgtgtc cgccgccagc     360

accaagggcc caagcgtgtt cccgctagcc cccagcagca agagcaccag cggcggcaca     420

gccgccctgg gctgcctggt gaaggactac ttccccgagc ccgtgaccgt gtcctggaac     480

agcggagccc tgacctccgg cgtgcacacc ttccccgccg tgctgcagag cagcggcctg     540

tacagcctga gcagcgtggt gaccgtgccc agcagcagcc tgggcaccca gacctacatc     600

tgtaacgtga accacaagcc cagcaacacc aaggtggaca agagagtgga gcccaagagc     660

tgtgacaaga cccacacctg ccccccctgc ccagcccccg agctgctggg cggacccagc     720

gtgttcctgt tcccccccaa gcccaaggac accctgatga tcagcagaac ccccgaggtg     780

acctgtgtgg tggtggacgt gtcccacgag gacccagagg tgaagttcaa ctggtacgtg     840

gacggcgtgg aggtgcacaa cgccaagacc aagcccagag aggagcagta caacagcacc     900

tacagggtgg tgtccgtgct gaccgtgctg caccaggact ggctgaacgg caaggagtac     960

aagtgtaagg tgtccaacaa ggccctgcca gccccaatcg aaaagaccat cagcaaggcc    1020

aagggccagc caagagagcc ccaggtgtac accctgccac ccagcaggga ggagatgacc    1080

aagaaccagg tgtccctgac ctgtctggtg aagggcttct acccaagcga catcgccgtg    1140

gagtgggaga gcaacggcca gcccgagaac aactacaaga ccaccccccc agtgctggac    1200

agcgacggca gcttcttcct gtacagcaag ctgaccgtgg acaagagcag atggcagcag    1260

ggcaacgtgt tcagctgctc cgtgatgcac gaggccctgc acaaccacta cacccagaag    1320

agcctgagcc tgtccccagg caag                                           1344

<210>  18
<211>  1335
<212>  DNA
<213>  Artificial sequence

<220>
<223>  Chimeric anti-CD151 antibody heavy chain derived from the
       murine 203B6 monoclonal antibody

<400>  18
gaagtgaaac tggtggagtc tggcggcgga ctggtgcagc caggcggcag cctgaagctg      60

tcctgcgccg ccagcggctt caccttcagc acctacacca tgagctgggt gcgccagacc     120

cccgagaagc ggctggaatg ggtggcctac atcagcagcg gcggagggac cacctactac     180

cccgacaccg tgaagggccg gttcaccatc agccgggaca acgcccggaa caccctgtac     240

ctgcagatga acagcctgaa gtccgaggac accgccatgt actactgcgc caccccccgg     300

atcggcaccg gcttcgccta ctggggccag ggcaccctgg tgaccgtgtc cgccgccagc     360

accaagggcc caagcgtgtt ccccctggcc ccctgctcca gaagcaccag cgagagcaca     420

gccgccctgg gctgcctggt gaaggactac ttccccgagc ccgtgaccgt gtcctggaac     480

agcggagccc tgaccagcgg cgtgcacacc ttccccgccg tgctgcagag cagcggcctg     540

tacagcctga gcagcgtggt gaccgtgccc agcagcagcc tgggcaccaa gacctacacc     600

tgtaacgtgg accacaagcc cagcaacacc aaggtggaca agagggtgga gagcaagtac     660

ggcccaccct gccccagctg cccagccccc gagttcctgg gcggacccag cgtgttcctg     720

ttccccccca agcccaagga caccctgatg atcagcagaa cccccgaggt gacctgtgtg     780

gtggtggacg tgtcccagga ggaccccgag gtccagttca actggtacgt ggacggcgtg     840

gaggtgcaca acgccaagac caagcccaga gaggagcagt ttaacagcac ctaccgggtg     900

gtgtccgtgc tgaccgtgct gcaccaggac tggctgaacg gcaaagagta caagtgtaag     960

gtctccaaca agggcctgcc aagcagcatc gaaaagacca tcagcaaggc caagggccag    1020

cctagagagc cccaggtcta caccctgcca cccagccaag aggagatgac caagaaccag    1080

gtgtccctga cctgtctggt gaagggcttc tacccaagcg acatcgccgt ggagtgggag    1140

agcaacggcc agcccgagaa caactacaag accacccccc cagtgctgga cagcgacggc    1200

agcttcttcc tgtacagcag gctgaccgtg gacaagtcca gatggcagga gggcaacgtc    1260

tttagctgct ccgtgatgca cgaggccctg cacaaccact acacccagaa gagcctgagc    1320

ctgtccctgg gcaag                                                      1335

<210>  19
<211>  6
<212>  PRT
<213>  mus musculus

<400>  19

Glu Asn Val Gly Thr Tyr
1               5

<210>  20
<211>  3
<212>  PRT
<213>  mus musculus

<400>  20

Gly Ala Ser
1


<210>  21
<211>  9
<212>  PRT
<213>  mus musculus

<400>  21

Gly Gln Thr Tyr Ser Phe Pro Tyr Thr
1               5


<210>  22
<211>  8
<212>  PRT
<213>  mus musculus

<400>  22

Gly Tyr Thr Phe Thr Ser Ser Ser
1               5


<210>  23
<211>  8
<212>  PRT
<213>  mus musculus

<400>  23

Ile His Pro Asn Ser Gly Asn Thr
1               5


<210>  24
<211>  12
<212>  PRT
<213>  mus musculus

<400>  24

Ala Arg Ala Arg Ser Phe Tyr Tyr Ala Met Asp Cys
1               5                   10


<210>  25
<211>  214
<212>  PRT
<213>  Artificial sequence

<220>
<223>  Chimeric anti-CD151 antibody light chain derived from the
       murine 214B2 monoclonal antibody

<400>  25

Asn Ile Val Met Thr Gln Ser Pro Lys Ser Met Ser Met Ser Val Gly

1               5                   10                  15

Glu Arg Val Thr Leu Ser Cys Lys Ala Ser Glu Asn Val Gly Thr Tyr
            20                  25                  30

Val Ser Trp Tyr Gln Gln Lys Pro Glu Gln Ser Pro Lys Leu Leu Ile
        35                  40                  45

Tyr Gly Ala Ser Asn Arg Tyr Thr Gly Val Pro Asp Arg Phe Thr Gly
    50                  55                  60

Ser Gly Ser Ala Thr Asp Phe Thr Leu Thr Ile Ser Ser Val Gln Ala
65                  70                  75                  80

Glu Asp Leu Ala Asp Tyr His Cys Gly Gln Thr Tyr Ser Phe Pro Tyr
                85                  90                  95

Thr Phe Gly Gly Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  26
<211>  449
<212>  PRT
<213>  Artificial sequence

<220>

<223>  Chimeric anti-CD151 antibody heavy chain derived from the
      murine 214B2 monoclonal antibody

<400>  26

Gln Val Gln Leu Gln Gln Pro Gly Ser Val Leu Val Arg Pro Gly Ala
1               5                   10                  15

Ser Val Lys Leu Ser Cys Lys Ala Ser Gly Tyr Thr Phe Thr Ser Ser
            20                  25                  30

Ser Met His Trp Ala Lys Gln Arg Pro Gly Gln Gly Leu Glu Trp Ile
        35                  40                  45

Gly Glu Ile His Pro Asn Ser Gly Asn Thr Asn Asn Asn Glu Lys Phe
    50                  55                  60

Lys Gly Lys Ala Thr Leu Thr Val Asp Thr Ser Ser Ser Thr Ala Tyr
65                  70                  75                  80

Val Asp Leu Ser Ser Leu Ser Ser Glu Asp Ser Ala Val Tyr Tyr Cys
                85                  90                  95

Ala Arg Ala Arg Ser Phe Tyr Tyr Ala Met Asp Cys Trp Gly Gln Gly
            100                 105                 110

Thr Ser Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Arg Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys

<210>  27

<211>  446
<212>  PRT
<213>  Artificial sequence

<220>
<223>  Chimeric anti-CD151 antibody heavy chain derived from the
       murine 214B2 monoclonal antibody

<400>  27

Gln Val Gln Leu Gln Gln Pro Gly Ser Val Leu Val Arg Pro Gly Ala
1               5                   10                  15

Ser Val Lys Leu Ser Cys Lys Ala Ser Gly Tyr Thr Phe Thr Ser Ser
            20                  25                  30

Ser Met His Trp Ala Lys Gln Arg Pro Gly Gln Gly Leu Glu Trp Ile
        35                  40                  45

Gly Glu Ile His Pro Asn Ser Gly Asn Thr Asn Asn Asn Glu Lys Phe
    50                  55                  60

Lys Gly Lys Ala Thr Leu Thr Val Asp Thr Ser Ser Ser Thr Ala Tyr
65                  70                  75                  80

Val Asp Leu Ser Ser Leu Ser Ser Glu Asp Ser Ala Val Tyr Tyr Cys
                85                  90                  95

Ala Arg Ala Arg Ser Phe Tyr Tyr Ala Met Asp Cys Trp Gly Gln Gly
            100                 105                 110

Thr Ser Val Thr Val Ser Ser Ala Ser Thr Lys Gly Pro Ser Val Phe
        115                 120                 125

Pro Leu Ala Pro Cys Ser Arg Ser Thr Ser Glu Ser Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Lys Thr Tyr Thr Cys Asn Val Asp His Lys Pro
        195                 200                 205

Ser Asn Thr Lys Val Asp Lys Arg Val Glu Ser Lys Tyr Gly Pro Pro
    210                 215                 220

Cys Pro Ser Cys Pro Ala Pro Glu Phe Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser Gln Glu Asp Pro Glu Val
            260                 265                 270

Gln Phe Asn Trp Tyr Val Asp Gly Val Glu Val His Asn Ala Lys Thr
        275                 280                 285

Lys Pro Arg Glu Glu Gln Phe Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Gly Leu Pro Ser Ser Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Gln Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Arg Leu Thr Val Asp Lys Ser Arg Trp
                405                 410                 415

Gln Glu Gly Asn Val Phe Ser Cys Ser Val Met His Glu Ala Leu His
            420                 425                 430

Asn His Tyr Thr Gln Lys Ser Leu Ser Leu Ser Leu Gly Lys
        435                 440                 445

<210>  28
<211>  18
<212>  DNA
<213>  mus musculus

<400>  28
gagaatgtgg gtacttat                                                    18


<210>  29
<211>  9
<212>  DNA
<213>  mus musculus

<400>  29
ggggcatcc                                                               9


<210>  30
<211>  27
<212>  DNA
<213>  mus musculus

<400>  30
ggacagactt acagctttcc gtacacg                                          27


<210>  31
<211>  24
<212>  DNA
<213>  mus musculus

<400>  31
ggctacacct tcaccagctc ctcg                                             24


<210>  32
<211>  24
<212>  DNA
<213>  mus musculus

<400>  32
attcatccta atagtggtaa tact                                             24


<210>  33
<211>  36
<212>  DNA
<213>  mus musculus

<400>  33
gcaagggcga ggtcctttta ctatgctatg gactgc                                36


<210>  34
<211>  642
<212>  DNA
<213>  Artificial sequence

<220>
<223>  Chimeric anti-CD151 antibody light chain derived from the

murine 214B2 monoclonal antibody

<400> 34
aacatcgtga tgacccagag ccccaagagc atgagcatga gcgtgggcga gagagtgacc 60

ctgagctgca aggccagcga gaacgtgggc acctacgtgt cctggtatca gcagaagccc 120

gagcagtccc ccaagctgct gatctacggc gccagcaacc ggtacaccgg cgtgcccgac 180

agattcaccg gcagcggcag cgccaccgac ttcaccctga ccatcagcag cgtgcaggcc 240

gaggacctgg ccgactacca ctgcggccag acctacagct tcccctacac ctttggcggc 300

ggaacaaagc tggaaatcaa gcgtacggtg gccgctccca gcgtgttcat cttcccccca 360

agcgacgagc agctgaagag cggcaccgcc agcgtggtgt gtctgctgaa caacttctac 420

cccagggagg ccaaggtgca gtggaaggtg gacaacgccc tgcagagcgg caacagccag 480

gagagcgtca ccgagcagga cagcaaggac tccacctaca gcctgagcag caccctgacc 540

ctgagcaagg ccgactacga gaagcacaag gtgtacgcct gtgaggtgac ccaccagggc 600

ctgtccagcc ccgtgaccaa gagcttcaac aggggcgagt gc 642

<210> 35
<211> 1347
<212> DNA
<213> Artificial sequence

<220>
<223> Chimeric anti-CD151 antibody heavy chain derived from the
      murine 214B2 monoclonal antibody

<400> 35
caggtgcagc tgcagcagcc cggcagcgtg ctggtgagac ctggcgcctc cgtgaagctg 60

tcctgcaagg cctccggcta caccttcacc agcagcagca tgcactgggc caagcagcgg 120

ccaggccagg gactggaatg gatcggcgag atccacccca acagcggcaa caccaacaac 180

aacgagaagt tcaagggcaa ggccaccctg accgtggaca ccagcagctc caccgcctac 240

gtggacctga gcagcctgtc cagcgaggac agcgccgtgt actactgcgc cagagccaga 300

tccttctact acgctatgga ctgctggggc cagggcacca gcgtgaccgt gtccagcgcc 360

agcaccaagg gcccaagcgt gttcccgcta gcccccagca gcaagagcac cagcggcggc 420

acagccgccc tgggctgcct ggtgaaggac tacttccccg agcccgtgac cgtgtcctgg 480

aacagcggag ccctgacctc cggcgtgcac accttccccg ccgtgctgca gagcagcggc 540

ctgtacagcc tgagcagcgt ggtgaccgtg cccagcagca gcctgggcac ccagacctac 600

atctgtaacg tgaaccacaa gcccagcaac accaaggtgg acaagagagt ggagcccaag 660

agctgtgaca agacccacac ctgccccccc tgcccagccc ccgagctgct gggcggaccc 720

agcgtgttcc tgttcccccc caagcccaag gacaccctga tgatcagcag aacccccgag 780

gtgacctgtg tggtggtgga cgtgtcccac gaggacccag aggtgaagtt caactggtac     840

gtggacggcg tggaggtgca caacgccaag accaagccca gagaggagca gtacaacagc     900

acctacaggg tggtgtccgt gctgaccgtg ctgcaccagg actggctgaa cggcaaggag     960

tacaagtgta aggtgtccaa caaggccctg ccagccccaa tcgaaaagac catcagcaag    1020

gccaagggcc agccaagaga gccccaggtg tacaccctgc cacccagcag ggaggagatg    1080

accaagaacc aggtgtccct gacctgtctg gtgaagggct tctacccaag cgacatcgcc    1140

gtggagtggg agagcaacgg ccagcccgag aacaactaca agaccacccc cccagtgctg    1200

gacagcgacg gcagcttctt cctgtacagc aagctgaccg tggacaagag cagatggcag    1260

cagggcaacg tgttcagctg ctccgtgatg cacgaggccc tgcacaacca ctacacccag    1320

aagagcctga gcctgtcccc aggcaag                                        1347

<210>  36
<211>  1338
<212>  DNA
<213>  Artificial sequence

<220>
<223>  Chimeric anti-CD151 antibody heavy chain derived from the
       murine 214B2 monoclonal antibody

<400>  36
caggtgcagc tgcagcagcc cggcagcgtg ctggtgagac ctggcgcctc cgtgaagctg      60

tcctgcaagg cctccggcta caccttcacc agcagcagca tgcactgggc caagcagcgg     120

ccaggccagg gactggaatg gatcggcgag atccacccca acagcggcaa caccaacaac     180

aacgagaagt tcaagggcaa ggccaccctg accgtggaca ccagcagctc caccgcctac     240

gtggacctga gcagcctgtc cagcgaggac agcgccgtgt actactgcgc cagagccaga     300

tccttctact acgctatgga ctgctggggc cagggcacca gcgtgaccgt gtccagcgcc     360

agcaccaagg gcccaagcgt gttccccctg gccccctgct ccagaagcac cagcgagagc     420

acagccgccc tgggctgcct ggtgaaggac tacttccccg agcccgtgac cgtgtcctgg     480

aacagcggag ccctgaccag cggcgtgcac accttccccg ccgtgctgca gagcagcggc     540

ctgtacagcc tgagcagcgt ggtgaccgtg cccagcagca gcctgggcac caagacctac     600

acctgtaacg tggaccacaa gcccagcaac accaaggtgg acaagagggt ggagagcaag     660

tacggcccac cctgccccag ctgcccagcc cccgagttcc tgggcggacc cagcgtgttc     720

ctgttccccc ccaagcccaa ggacaccctg atgatcagca gaacccccga ggtgacctgt     780

gtggtggtgg acgtgtccca ggaggacccc gaggtccagt tcaactggta cgtggacggc     840

gtggaggtgc acaacgccaa gaccaagccc agagaggagc agtttaacag cacctaccgg     900

gtggtgtccg tgctgaccgt gctgcaccag gactggctga acggcaaaga gtacaagtgt 960

aaggtctcca acaagggcct gccaagcagc atcgaaaaga ccatcagcaa ggccaagggc 1020

cagcctagag agccccaggt ctacaccctg ccacccagcc aagaggagat gaccaagaac 1080

caggtgtccc tgacctgtct ggtgaagggc ttctacccaa gcgacatcgc cgtggagtgg 1140

gagagcaacg gccagcccga gaacaactac aagaccaccc ccccagtgct ggacagcgac 1200

ggcagcttct tcctgtacag caggctgacc gtggacaagt ccagatggca ggagggcaac 1260

gtctttagct gctccgtgat gcacgaggcc ctgcacaacc actacaccca gaagagcctg 1320

agcctgtccc tgggcaag 1338

## Description

The present invention relates to new antibodies, especially monoclonal antibodies of murine origin, which are chimeric and humanised, and which are capable of inhibiting tumour growth, and also to the amino acid and nucleic sequences coding for those antibodies. According to a particular aspect, the invention relates to new antibodies, derivative compounds or functional fragments, which are capable of inhibiting the proliferation of tumour cells. The invention also includes use of those antibodies as medicaments for the prophylactic and/or therapeutic treatment of cancers and also in cancer diagnostic methods or kits. Finally, the invention includes products and/or compositions comprising such antibodies in association, for example, with anti-cancer agents and/or antibodies or conjugated with toxins, and use thereof in the prevention and/or treatment of certain cancers.

CD151, also referred to as PETA-3 or SFA-1, is a membrane protein belonging to the tetraspanin family (Boucheix and Rubinstein, 2001, Cell Mol. Life Sci. 58, 1189-1205; Hemler, 2001, J. Cell Biol. 155, 1103-1107). In humans, CD151 has 253 amino acids and includes 4 membrane fragments and 2 extracellular domains EC1 (18 amino acids, sequence [40-57]) and EC2 (109 amino acids, sequence [113-221]) which are also referred to as extracellular loops. It is to be noted, however, that, in the nucleotide sequence, two variants of CD151 have been identified hitherto, namely one having nucleotides A and C at positions 395 and 409, respectively, [Fitter et al., 1995, Blood 86(4), 1348-1355] and the other having, at the same positions, nucleotides G and T instead of nucleotides A and C [Hasegawa et al., 1996, J. Virol. 70(5), 3258-3263]. As a result, a mutation can be observed in the peptide sequence, namely a mutation of the residues K (Lys) and P (Pro) at positions 132 and 137, respectively, to the residues R (Arg) and S (Ser) [Fitter et al., 1995, Blood 86(4), 1348-1355 / Hasegawa et al., 1996, J. Virol. 70(5), 3258-3263].

CD151 is overexpressed in numerous cancers such as, for example, cancers of the lung [Tokuhara et al., 2001, Clin. Cancer Res. 7, 4109-4114], colon [Hashida et al., 2003, Br. J. Cancer 89, 158-167], prostate [Ang et al., 2004, Cancer Epidemiol. Biomarkers Prev. 13, 1717-1721] or pancreas [Gesierich et al., 2005, Clin. Cancer Res. 11,2840-2852].

The use of knock-out mice which do not express CD151 and of anti-CD151 antibodies and siRNA in order to block, *in vitro,* the functionality and expression of CD151 in various types of cell has allowed it to be shown that CD151 is involved in a number of phenomena related to cancer, such as cell adhesion (Nishiuchi et al., 2005, Proc. Natl. Acad. Sci. USA 102, 1939-1944; Winterwood et al., 2006, Mol. Biol. Cell 17, 2707-2721), cell motility (Kohno et al., 2002, Int. J. Cancer 97, 336-343), cell migration (Yauch et al., 1998, Mol. Biol. Cell 9, 2751-2765; Testa et al., 1999, Cancer Res. 59, 3812-3820; Penas et al., 2000, J. Invest. Dermatol. 114, 1126-1135; Klosek et al., 2005, Biochem. Biophys. Res. Commun. 336, 408-416), cell invasion (Kohno et al., 2002, Int. J. Cancer 97, 336-343; Shiomi et al., 2005, Lab. Invest. 85, 1489-1506; Hong et al., 2006, J. Biol. Chem. 281, 24279-24292) and angiogenesis (Yanez-Mo et al., 1998, J. Cell Biol. 141, 791-804; Sincock et al., 1999, J. Cell Sci. 112, 833-844; Takeda et al., 2007, Blood 109, 1524-1532).

One of the noteworthy properties of the tetraspanins is their ability to form associations amongst themselves and also with a large number of other surface molecules so as to form structured macromolecular complexes. Within those complexes, each tetraspanin is associated specifically with one or more surface molecules, thereby forming primary complexes composed of a tetraspanin and a partner molecule. The tetraspanins are capable of organising particular microdomains of the plasma membrane from which microdomains they may recruit their partner molecules, which may be functionally coupled. The set of interactions involving the tetraspanins has been referred to as the "network of tetraspanins" or "Tetraspanin Web".

CD151 interacts on the surface of cells with various membrane proteins. In particular, there have been identified highly stable complexes, resistant to the action of certain detergents, with laminin receptor integrins, more particularly with the integrins α3β1or α6β4, whose preferred ligand is laminin 5 (Yauch et al., 1998, Mol. Biol. Cell 9, 2751-2765; Lammerding et al., 2003, Proc. Natl. Acad. Sci USA 100, 7616-7621). This association involves the extracellular domains of CD151 and the integrins. The sequence QRD [194-196] of CD151, located in the EC2 loop, is very important in that association because mutation of this site causes loss of interaction with certain integrins (Kazarov et al., 2002, J. Cell Biol. 158, 1299-1309). Functional ternary complexes of CD151/integrin α6β4/c-Met (HGF receptor) have moreover been identified in tumour cells (Klosek et al., 2005, Biochem. Biophys. Res. Commun. 336, 408-416). Inhibition of the expression of CD151 as a result of treating cells with an interference RNA results in inhibition of the cell growth and migration caused by HGF.

The interactions, within a particular cell, between CD151 and other tetraspanins, necessary for formation of the network of tetraspanins, are thought to depend on the membrane and cytoplasmic regions of CD151 because it has been shown that deletion of the EC2 loop does not disrupt the association of CD151 with other tetraspanins (Berditchevski, 2001, J. Cell Sci. 114, 4143-4151).

CD151 is capable of regulating the phenomena of cell adhesion, migration and invasion by modulation of various signalling pathways such as, for example, the phosphoinositide pathway *via* an association with PI4-kinase (Yauch et al., 1998, Mol. Biol. Cell 9, 2751-2765), the c-Jun signalling pathway *via* the phosphorylation of FAK, Src, p38-MAPK and JNK (Hong et al., 2006, J. Biol. Chem. 281, 24279-24292), the phosphorylation of integrins by PKC (Zhang et al., 2001, J. Biol. Chem. 276, 25005-25013) and the activation of GTPases of the Rho family (Shigeta et al., 2003, J. Cell Biol. 163, 165-176).

Homophilic-type interactions between cells are also responsible for an increase in cell motility and in expression of the metalloproteinase MMP-9 (Hong et al., 2006, J. Biol. Chem. 281, 24279-24292). Those intercellular CD151-CD151 interactions bring about the activation of c-Jun *via* the phosphorylation of FAK, Src, p38-MAPK and JNK.

Despite the interest in the CD151 protein, two therapeutically aimed antibodies have been generated to date, namely the monoclonal antibodies 50-6 and SFA1.2B4. These 2 antibodies have comparable activities. Although they do inhibit the formation of metastases *in vivo* in animal models, no effect on tumour growth *in vivo* has been established.

The monoclonal antibody 50-6 (IgG1 isotype) directed to CD151 was generated in the mouse by subtractive immunisations with human epidermoid carcinoma HEp-3 cells (Testa et al., 1999, Cancer Res. 59, 3812-3820).

The antibody 50-6 is capable of inhibiting, *in vitro,* migration of human cervical carcinoma HeLa cells, transfected so as to overexpress CD151, and of HEp-3 cells and angiogenesis in a model of chorio-allantoic membrane neovascularisation caused by bFGF (basic fibroblast growth factor). *In vivo* it inhibits the metastases brought about by inoculation of HEp-3 cells in 2 chicken embryo models (Testa et al., 1999, Cancer Res. 59, 3812-3820). In these models, the inhibitory activity of the antibody 50-6 is determined by measurement of the activity of the protein huPA (human urokinase-type plasminogen activator) in lung extracts. According to the authors, this assay reflects the presence of human cells in the lungs. After assaying, the reduction in metastases (dissemination of HEp-3 cells into the chicken embryo lungs) that is brought about by the antibody 50-6 is estimated, by comparison with a control antibody, to be 74% in a so-called "spontaneous metastasis" model, in which inoculation of the cells is followed by injection of the antibody, and 57% in a so-called "experimental metastasis" model, in which the cells and the antibody are inoculated together. According to the authors, the anti-tumour properties of the antibody 50-6 that are observed *in vivo* do not seem to be related to a cytostatic or cytotoxic effect because the antibody showed no effect on the *in vitro* proliferation of HEp-3 cells.

The hybridoma producing the antibody 50-6 is available at the ATCC under the reference CRL-2696 (hybridoma initially deposited under the reference 50-6 [PTA-227]).

The anti-CD151 monoclonal antibody SFA1.2B4 (IgG1 isotype) was generated in the mouse after immunisation by the intraperitoneal route with NIH 3T3 cells transfected by the human CD151 gene (Hasegawa et al., 1996, J. Virol. 70, 3258-3263). The antibody SFA1.2B4 is capable of inhibiting *in vitro* cell invasion and motility of various human tumour lines (Kohno et al., 2002, Int. J. Cancer 97, 336-343). It inhibits *in vivo* the pulmonary metastases caused by colon cancer RPMI 14788 and fibrosarcoma HT1080 lines transfected so as to overexpress CD151 (Kohno et al., 2002, Int. J. Cancer 97, 336-343).

Other murine anti-CD151 antibodies have been described in the literature, such as, for example, the monoclonal antibodies 14A2H1 (Ashman et al., 1991, Br. J. Haematol. 79, 263-270; Roberts et al., 1995, Br. J. Haematol. 89, 853-860), TS151 and TS151R (Serru et al., 1999, Biochem. J. 340, 103-111; Geary et al., 2001, Tissue Antigens 58, 141-153; Charrin et al., J. Biol. Chem. 276, 14329-14337; Chometon et al., 2006, Exp. Cell Res. 312, 983-985).

Several experimental studies have shown the major role of the tetraspanins in the formation of metastases by acting either as suppressors or as promoters of metastases. Accordingly, the transfection of tetraspanins such as CD9, CD63 or CD82 reduces the metastatic potential of cancer lines. In contrast, expression of the tetraspanins CD151 and Co-029 seems to produce the opposite effect. These 2 tetraspanins are therefore thought to be promoters of metastasis. These results are consistent with various clinical studies which have shown that, in a number of cancers (breast, lung, oesophagus, stomach, liver, pancreas, colon, prostate, melanoma, ...), CD9 and CD82 are less expressed in primary tumours when there is metastasis and that a reduction in their expression is predictive of a lower survival rate. In lung cancer, the combined reduction in the expression of CD9 and CD82 has been correlated with greater metastatic potential than when expression of just one of those two antigens is reduced.

Several retrospective studies have shown that overexpression of CD151 is associated with aggressiveness of certain cancers, such as lung, colon and prostate cancers, and that it might be considered to be a factor for poor prognosis (Tokuhara et al., 2001, Clin. Cancer Res. 7, 4109-4114; Hashida et al., 2003, Br. J. Cancer 89, 158-167; Ang et al., 2004, Cancer Epidemiol. Biomarkers Prev. 13, 1717-1721). In these cases, mean survival is in fact reduced in those patients having tumours which express CD151, compared to those having tumours which do not express CD151.

The overexpression of CD151 in various human tumour lines (HeLa, RPMI14788, A172, HT1080), brought about by transfection of the corresponding gene, causes an increase in motility, migration and invasion of the transfected cells (Testa et al., 1999, Cancer Res. 59, 3812-3820; Kohno et al., 2002, Int. J. Cancer 97, 336-343). These phenomena are inhibited in the presence of anti-CD151 antibodies.

A chimeric (also written chimearic) antibody is understood as referring to an antibody which contains a natural variable (light chain and heavy chain) region derived from an antibody from a given species in association with the constant light chain and heavy chain regions of an antibody from a heterologous species to said given species.

Chimeric-type antibodies, or their fragments, according to the invention can be prepared using genetic recombination techniques. For example, the chimeric antibody may be produced by cloning a recombinant DNA comprising a promoter and a sequence coding for the variable region of a non-human, especially murine, monoclonal antibody according to the invention and a sequence coding for the constant region of a human antibody. A chimeric antibody of the invention encoded by such a recombinant gene may be, for example, a mouse-human chimaera, the specificity of that antibody being determined by the variable region derived from the murine DNA and its isotype determined by the constant region derived from the human DNA. For methods of preparing chimeric antibodies, reference may be made, for example to the document Verhoeyn et al. (BioEssays, 8:74, 1988).

In accordance with a first aspect, the invention relates to a chimeric antibody, or a derived compound or functional fragment, **characterised in that** it comprises a light chain having a sequence comprising the amino acid sequence SEQ ID No. 7 or having at least 80%, preferably 85%, 90%, 95% and 98%, identity, after optimal alignment, with the sequence SEQ ID No. 7, and/or comprises a heavy chain having a sequence comprising the amino acid sequence SEQ ID No. 8 or 9 or having at least 80%, preferably 85%, 90%, 95% and 98%, identity, after optimal alignment, with the sequence SEQ ID No. 8 or 9.

The two heavy chain sequences, SEQ ID Nos. 8 and 9, are corresponding to the human isotype IgG1 and IgG4, respectively.

As a consequence, a first embodiment of the invention discloses a chimeric antibody c203B6[IgG1], or a derived compound or functional fragment of same, of the invention comprises a light chain sequence comprising the amino acid sequence SEQ ID No. 7, and in that it comprises a heavy chain sequence comprising the amino acid sequence SEQ ID No. 8.

A second embodiment of the invention discloses a chimeric antibody c203B6[IgG4], or a derived compound or functional fragment of same, of the invention comprises a light chain sequence comprising the amino acid sequence SEQ ID No. 7, and in that it comprises a heavy chain sequence comprising the amino acid sequence SEQ ID No. 9.

In another preferred embodiment, the chimeric antibody, or a derived compound or functional fragment, of the invention is **characterised in that** it comprises a light chain having a sequence comprising the amino acid sequence SEQ ID No. 25 or having at least 80%, preferably 85%, 90%, 95% and 98%, identity, after optimal alignment, with the sequence SEQ ID No. 25, and/or comprises a heavy chain having a sequence comprising the amino acid sequence SEQ ID No. 26 or 27 or having at least 80%, preferably 85%, 90%, 95% and 98%, identity, after optimal alignment, with the sequence SEQ ID No. 26 or 27.

The two heavy chain sequences, SEQ ID Nos. 26 and 27, are corresponding to the human isotype IgG1 and IgG4, respectively.

As a consequence, a first embodiment of the invention discloses a chimeric antibody c214B2[IgG1], or a derived compound or functional fragment of same, of the invention comprises a light chain sequence comprising the amino acid sequence SEQ ID No. 25, and in that it comprises a heavy chain sequence comprising the amino acid sequence SEQ ID No. 26.

A second embodiment of the invention discloses a chimeric antibody c214B2[IgG4], or a derived compound or functional fragment of same, of the invention comprises a light chain sequence comprising the amino acid sequence SEQ ID No. 25, and in that it comprises a heavy chain sequence comprising the amino acid sequence SEQ ID No. 27.

A "functional fragment" of an antibody according to the invention is understood to refer to, especially, an antibody fragment, such as Fv, scFv (sc standing for "single chain"), Fab, F(ab')₂, Fab' or scFv-Fc fragments or diabodies, or any fragment whose half-life may have been increased. Such functional fragments will be described in detail hereinbelow in the present description.

A "derivative compound" of an antibody according to the invention is understood to denote, especially, a binding protein comprising a peptide framework or "scaffold" and at least one of the CDRs of the original antibody in order to preserve its recognition ability. Such derivative compounds are well known to the person skilled in the art and will be described in greater detail hereinbelow in the present description.

More preferably, the invention includes the antibodies, their derivative compounds or their functional fragments, according to the present invention, which are especially chimeric or humanised, that are obtained by genetic recombination or by chemical synthesis.

According to a preferred embodiment, the antibody, or one of its derivative compounds or functional fragments, according to the invention is **characterised in that** it consists of a monoclonal antibody.

A "monoclonal antibody" is to be understood as an antibody derived from a population of substantially homogeneous antibodies. More especially, the individual antibodies of a population are identical with the exception of a few possible mutations that may be produced naturally and that may be present in minimal amounts. In other words, a monoclonal antibody consists of a homogeneous antibody resulting from the proliferation of just one cell clone (for example, a hybridoma, a eukaryotic host cell transfected with a DNA molecule coding for the homogeneous antibody, a prokaryotic host cell transfected with a DNA molecule coding for the homogeneous antibody, etc.) and which is usually characterised by heavy chains of one and the same class and subclass and light chains of just one type. Monoclonal antibodies are highly specific and are directed to a single antigen. In addition, in contrast to preparations of polyclonal antibodies which customarily comprise different antibodies directed to different determinants, or epitopes, each monoclonal antibody is directed to a single epitope of the antigen.

It must be understood herein that the invention does not relate to the antibodies in natural form, that is to say they are not taken from their natural environment but rather it has been possible to isolate them or obtain them by purification starting from natural sources, or else obtain them by genetic recombination, or by chemical synthesis, and they may accordingly contain non-natural amino acids as will be described hereinbelow.

The IMGT unique numbering system was defined so as to compare variable domains whatever the antigen, chain type or species [Lefranc M.-P., Immunology Today 18, 509 (1997); Lefranc M.-P., The Immunologist, 7, 132-136 (1999); Lefranc, M.-P., Pommié, C., Ruiz, M., Giudicelli, V., Foulquier, E., Truong, L., Thouvenin-Contet, V. and Lefranc, Dev. Comp. Immunol., 27, 55-77 (2003)]. In this numbering system, the conserved amino acids always retain the same position, such as cysteine 23 (1st-CYS), tryptophan 41 (CONSERVED TRP), the hydrophobic amino acid 89, cysteine 104 (2nd-CYS), phenylalanine or tryptophan 118 (J-PHE or TRP). The IMGT unique numbering system accordingly provides standardised delimitation of the scaffold regions (FR1-IMGT: positions 1 to 26, FR2-IMGT: positions 39 to 55, FR3-IMGT: positions 66 to 104 and FR4_IMGT: positions 118 to 128) and also of the complementarity determining regions or CDRs (CDR1-IMGT: positions 27 to 38, CDR2-IMGT: positions 56 to 65 and CDR3-IMGT: positions 105 to 117). As the "holes" or "spaces" represent unoccupied positions, the lengths of the CDRs according to IMGT become crucial information. The IMGT system is used in 2D graphical representations which are then referred to as IMGT pearl necklaces [Ruiz, M. and Lefranc, M.-P., Immunogenetics, 53, 857-883 (2002); Kaas, Q. and Lefranc, M.-P., Current Bioinformatics, 2, 21-30 (2007)] and in 3D structures referred to as IMGT/3Dstructure-DB [Kaas, Q., Ruiz, M. and Lefranc, M.-P., T cell receptor and MHC structural data. Nucl. Acids. Res., 32, D208-D210 (2004)].

In the present description, the terms "polypeptides", "polypeptide sequences", "peptides" and "proteins" associated with the antibody compounds or their sequences are interchangeable.

It must be understood herein that the invention does not relate to the antibodies in natural form, that is to say they are not taken from their natural environment but may have been isolated or obtained by purification starting from natural sources, or else obtained by genetic recombination, or by chemical synthesis, and they may accordingly contain non-natural amino acids as will be described hereinbelow.

"Percentage identity" between two nucleic acid or amino acid sequences is understood by the present invention to denote the percentage of nucleotides or amino acid residues that are identical between the two sequences being compared, obtained after the best alignment (optimal alignment), this percentage being purely statistical and the differences between the two sequences being randomly distributed throughout their length. Sequence comparisons between two nucleic acid or amino acid sequences are customarily carried out by comparing those sequences after they have been optimally aligned, it being possible for said comparison to be carried out segment by segment or by means of a "comparison window". The optimal alignment of sequences for the comparison can, besides being carried out manually, be carried out by means of the local homology algorithm of Smith and Waterman (1981) [Ad. App. Math. 2:482], by means of the local homology algorithm of Neddleman and Wunsch (1970) [J. Mol. Biol. 48:443], by means of the similarity search method of Pearson and Lipman (1988) [Proc. Natl. Acad. Sci. USA 85:2444], or by means of computer software employing those algorithms (GAP, BESTFIT, FASTA and TFASTA in the Wisconsin Genetics Software Package, Genetics Computer Group, 575 Science Dr., Madison, WI, or by the comparison software BLAST N or BLAST P).

The percentage identity between two nucleic acid or amino acid sequences is determined by comparing the two optimally aligned sequences in which the nucleic acid or amino acid sequence to be compared can contain additions or deletions with respect to the reference sequence for optimal alignment between those two sequences. The percentage identity is calculated by determining the number of identical positions where the nucleotide or amino acid residue is identical between the two sequences, dividing that number of identical positions by the total number of positions in the comparison window and multiplying the result obtained by 100 in order to obtain the percentage identity between the two sequences.

For example, there may be used the BLAST program "BLAST 2 sequences" (Tatusova et al., "Blast 2 sequences - a new tool for comparing protein and nucleotide sequences", FEMS Microbiol Lett. 174:247-250) available on the website http://www.ncbi.nlm.nih.gov/gorf/b12.html, the parameters used being those given as default (especially for the parameters "open gap penalty": 5, and "extension gap penalty": 2; the selected matrix being, for example, the "BLOSUM 62" matrix suggested by the program), the percentage identity between the two sequences for comparison being calculated directly by the program.

As an amino acid sequence having at least 80%, preferably 85%, 90%, 95% and 98%, identity with a reference amino acid sequence preference is given to one having, with respect to the reference sequence, certain modifications, especially a deletion, addition or substitution of at least one amino acid, a truncation or an extension. In the case of a substitution of one or more consecutive or non-consecutive amino acid(s) preference is given to substitutions in which the substituted amino acids are replaced by "equivalent" amino acids. The expression "equivalent amino acids" is intended herein to denote any amino acid capable of being substituted for one of the amino acids of the basic structure without, however, fundamentally modifying the biological activities of the corresponding antibodies, such as will be defined hereinbelow, especially in the Examples.

These equivalent amino acids can be determined either on the basis of their structural homology with the amino acids for which they are being substituted or on the basis of results of comparative biological activity tests between the various antibodies that may be produced.

By way of non-limiting example, Table 1 below recalls the substitution possibilities capable of being carried out without resulting in fundamental modification of the biological activity of the corresponding modified antibody, the reverse substitutions being feasible of course under the same conditions.

**Table 1**

| **Original residue** | **Substitution(s)** |
|---|---|
| Ala (A) | Val, Gly, Pro |
| Arg (R) | Lys, His |
| Asn (N) | Gln |
| Asp (D) | Glu |
| Cys (C) | Ser |
| Gln (Q) | Asn |
| Glu (G) | Asp |
| Gly (G) | Ala |
| His (H) | Arg |
| Ile (I) | Leu |
| Leu (L) | Ile, Val, Met |
| Lys (K) | Arg |
| Met (M) | Leu |
| Phe (F) | Tyr |
| Pro (P) | Ala |
| Ser (S) | Thr, Cys |
| Thr (T) | Ser |
| Trp (W) | Tyr |
| Tyr (Y) | Phe, Trp |
| Val (V) | Leu, Ala |

As indicated hereinbefore, the invention is likewise directed at any compound derived from an antibody according to the invention.

More especially, the antibody, or one of its derivative compounds or functional fragments, according to the invention is **characterised in that** said derivative compound consists of a binding protein comprising a peptide scaffold onto which is grafted at least one CDR so as to preserve, in entirety or in part, the paratopic recognition properties of the initial antibody.

One or more sequences among the sequences of the CDRs described in this invention can also be provided on various protein scaffolds - or frameworks - of immunoglobulins. In this case, the protein sequence makes it possible to recreate a peptide skeleton favourable to the folding of the grafted CDR or CDRs, allowing it/them to preserve their paratopic properties of antigen recognition.

In general manner, the person skilled in the art will know how to determine the type of protein scaffold onto which to graft at least one of the CDRs derived from the original antibody. More especially, it is known that, in order to be selected, such scaffolds must meet the greatest number of criteria as listed below (Skerra A., J. Mol. Recogn. 13, 2000, 167-187):
- good phylogenetic conservation;
- known three-dimensional structure (such as, for example, from crystallography, NMR spectroscopy or any other technique known to the person skilled in the art);
- small size;
- few or no post-transcriptional modifications; and/or
- ease of production, expression and purification.

The origin of such protein scaffolds can be, but is not limited to, structures selected from: fibronectin and preferably the 10th domain of type 3 fibronectin, lipocalin, anticalin (Skerra A., J. Biotechnol., 2001, 74(4) :257-75), protein Z derived from domain B of protein A of *Staphylococcus aureus,* thioredoxin A and also proteins having repeated motifs of the "ankyrin repeat" (Kohl et al., PNAS, 2003, vol.100, No. 4, 1700-1705), "armadillo repeat", "leucine-rich repeat" or "tetratricopeptide repeat" types.

There may also be mentioned scaffolds derived from toxins such as, for example, the following toxins derived from scorpions, insects, plants, molluscs, etc. or protein inhibitors of neuronal NO synthase (PIN).

As an example - which is in no way limiting - of such hybrid structures there may be mentioned the insertion of the CDR-H1 (heavy chain) of an anti-CD4 antibody, namely 13B8.2, into one of the loops of PIN, the new binding protein thereby obtained retaining the same binding properties as the original antibody (Bes et al., BBRC 343, 2006, 334-344). There may also be mentioned, by way of illustration, the grafting of the CDR-H3 (heavy chain) of an anti-lysozyme VHH antibody onto one of the loops of neocarzinostatin (Nicaise *et al.,* 2004).

Finally, as described hereinbefore, such peptide scaffolds can comprise from 1 to 6 CDR(s) derived from the original antibody. Preferably, but without its being a necessity, the person skilled in the art will select at least one CDR derived from the heavy chain, the latter being known to be principally responsible for the specificity of the antibody. Selection of the relevant CDR(s) will be obvious to the person skilled in the art, the latter employing known techniques for the purpose (Bes et al., FEBS letters 508, 2001 67-74).

Obviously, these examples are in no way limiting, and any other structure known or obvious to the skilled person must be considered as being included within the protection afforded by the present patent application.

The present invention accordingly relates to an antibody, or one of its derivative compounds or functional fragments, **characterised in that** said peptide scaffold is selected from proteins which a) are phylogenetically well conserved, b) are of robust architecture, c) have well-known three-dimensional molecular organisation, d) are of small size and/or e) comprise regions that can be modified by deletion and/or insertion without changes to the stability properties.

According to a preferred embodiment, the antibody, or one of its derivative compounds or functional fragments, according to the invention is **characterised in that** the peptide scaffold is selected from i) scaffolds derived from fibronectin, preferably the 10th domain of type 3 fibronectin, lipocalin, anticalin, protein Z derived from domain B of protein A of *Staphylococcus aureus,* thioredoxin A, ii) proteins having repeated motifs of the "ankyrin repeat", "armadillo repeat", "leucine-rich repeat" or "tetratricopeptide repeat" type and also iii) protein inhibitors of neuronal NO synthase (PIN).

In accordance with another aspect of the invention, mention is likewise made of the functional fragments of the antibody described hereinbefore.

More especially, the invention is directed to an antibody, or one of its derivative compounds or functional fragments, the invention **characterised in that** said functional fragment is selected from Fv, Fab, (Fab')₂, Fab', scFv and scFv-Fc fragments and diabodies, and any fragment whose half-life may have been extended such as pegylated fragments.

Such functional fragments of antibodies according to the invention consist, for example, of Fv, scFv (sc standing for single chain), Fab, F(ab')₂, Fab' or scFv-Fc fragments or diabodies, or any fragment whose half-life may have been extended by chemical modification, e.g. addition of poly(alkylene)glycol such as poly(ethylene)glycol ("PEGylation") (the PEGylated fragments being referred to as Fv-PEG, scFv-PEG, Fab-PEG, F(ab')₂-PEG or Fab'-PEG) ("PEG" from the designation Poly(Ethylene)Glycol), or by incorporation in a liposome, microspheres or PLGA, said fragments having at least one of the characteristic CDRs according to the invention and, especially, being capable of generally exerting activity, even partial, of the antibody from which it is derived.

Preferably, said functional fragments will be composed of or will comprise a partial sequence of the variable heavy or light chain of the antibody from which they are derived, said partial sequence being sufficient to retain the same binding specificity as the antibody from which it is derived and an adequate affinity, preferably equal to at least 1/100th, more preferably at least 1/10th, of that of the antibody from which it is derived.

Such a functional fragment will comprise at least 5 consecutive amino acids, preferably 10, 15, 25, 50 or 100 consecutive amino acids, from the sequence of the antibody from which it is derived.

Also preferred are functional fragments which comprise at least:
- the 3 CDRs of the light chain comprised in the SEQ ID No. 7 and 112 consecutive amino acids, preferably 115, 125, 175, 200 or 210 consecutive amino acids, from the sequence SEQ ID No. 7; and/or
- the 3 CDRs of the heavy chain comprised in the SEQ ID No. 8 or 9 and 119 consecutive amino acids, preferably 125, 150, 200, 250 or 300 consecutive amino acids, from the sequence SEQ ID No. 8 or 9.

Are also preferred functional fragments which comprise at least:
- the 3 CDRs of the light chain comprised in the SEQ ID No. 25 and 108 consecutive amino acids, preferably 115, 125, 175, 200 or 210 consecutive amino acids, from the sequence SEQ ID No. 25; and/or
- the 3 CDRs of the heavy chain comprised in the SEQ ID No. 26 or 27 and 120 consecutive amino acids, preferably 125, 150, 200, 250 or 300 consecutive amino acids, from the sequence SEQ ID No. 26 or 27.

Preferably, these functional fragments will be fragments of Fv, scFv, Fab, F(ab')₂, F(ab'), scFv-Fc type, or diabodies, which generally have the same fixing specificity as the antibody from which they are obtained. According to the present invention, fragments of antibodies of the invention can be obtained starting from antibodies as described hereinbefore by methods such as digestion using enzymes such as pepsin or papain and/or by cleavage of the disulfide bridges by means of chemical reduction. The antibody fragments included in the present invention can also be obtained by genetic recombination techniques that are likewise well-known to the person skilled in the art or by peptide synthesis by means of, for example, automatic peptide synthesisers such as those supplied by the company Applied Biosystems, etc..

According to another particular aspect, the present invention relates to a chimeric antibody, or one of its derivative compounds or functional fragments, according to the invention, **characterised in that** said antibody also comprises constant light chain and heavy chain regions derived from an antibody from a species heterologous to the mouse, especially from humans.

According to yet another aspect of the invention, the humanised antibody, or one of its derivative compounds or functional fragments, is **characterised in that** the constant light chain and heavy chain regions derived from a human antibody are the lambda or kappa region and the gammy-1, gamma-2 or gamma-4 region, respectively.

According to another aspect, the invention relates to a first murine hybridoma from which the chimeric monoclonal antibodies c203B6[IgG1] and c203B6[IgG4] according to the present invention are derived, especially the hybridoma of murine origin as deposited at the Centre National de Cultures de Microorganismes (CNCM) (Institut Pasteur, Paris, France) on 22nd February 2008 under number I-3920. Said hybridoma was obtained by fusion of immunised Balb/c mice splenocytes and Sp 20 Ag 14 myeloma cell lines.

Finally, according to another aspect, the invention relates to a second murine hybridoma from which the chimeric monoclonal antibodies c214B2[IgG1] and c214B2[IgG4] according to the present invention are derived, especially the hybridoma of murine origin as deposited at the Centre National de Cultures de Microorganismes (CNCM) (Institut Pasteur, Paris, France) on 21st February 2008 under number I-3919. Said hybridoma was obtained by fusion of immunised Balb/c mice splenocytes and Sp 20 Ag 14 myeloma cell lines.

Table 2 hereinbelow summarises the different amino acid sequences corresponding to the different antibodies according to the invention with, for information, the CDR sequences defined according to IMGT and the chimeric light and heavy chains.

**Table 2**

| | c203B6 | c214B2 |
|---|---|---|
| CDR-L1 | 1 | 19 |
| CDR-L2 | 2 | 20 |
| CDR-L3 | 3 | 21 |
| Chim. VL | 7 | 25 |
| CDR-H1 | 4 | 22 |
| CDR-H2 | 5 | 23 |
| CDR-H3 | 6 | 24 |
| IgG1 Chim. VH | 8 | 26 |
| IgG4 Chim. VH | 9 | 27 |

Antibodies according to the present invention also include humanised antibodies.

A humanised antibody is understood as referring to an antibody which contains CDR regions derived from an antibody of non-human origin, the other parts of the antibody molecule being derived from one (or more) human antibody/antibodies or germline(s). In addition, some of the residues of the segments of the skeleton (referred to as FR) can be modified in order to preserve the binding affinity (Jones et al., Nature, 321:522-525, 1986; Verhoeyen et al., Science, 239:1534-1536, 1988; Riechmann et al., Nature, 332:323-327, 1988).

The humanised antibodies according to the invention or fragments thereof can be prepared by techniques known to the person skilled in the art (such as, for example, those described in the documents Singer et al., J. Immun. 150:2844-2857, 1992; Mountain et al., Biotechnol. Genet. Eng. Rev., 10:1-142, 1992; or Bebbington et al., Bio/Technology, 10:169-175, 1992). Such humanised antibodies according to the invention are preferred for their use in *in vitro* diagnostic methods or in *in vivo* prophylactic and/or therapeutic treatment. Other humanisation techniques are also known to the person skilled in the art, such as, for example, the technique of "*CDR Grafting*", described by PDL, which is the subject-matter of patents EP 0 451 261, EP 0 682 040, EP 0 939 127, EP 0 566 647 or also US 5,530,101, US 6,180,370, US 5,585,089 and US 5,693,761. There may also be mentioned the patents US 5,639,641 or also 6,054,297, 5,886,152 and 5,877,293.

In addition, the invention is also directed at the humanised antibodies derived from the chimeric antibodies described hereinbefore.

Preferably, the constant light chain and heavy chain regions derived from a human antibody are the lambda or kappa region and the gamma-1, gamma-2 or gamma-4 region, respectively.

IgG4-derived antibodies can be further modified as described by Angal et al., 1993, in order to stabilize the hinge region leading to the expression of more homogenous antibodies (Ser226 to Pro modification).

In the embodiment corresponding to the IgG1 isotype, an additional characteristic of the antibody is that of having effector functions such as ADCC (Antibody Dependent Cellular Cytotoxicity) and/or CDC (Complement Dependent Cytotoxicity).

In addition, as will emerge from the Examples hereinbelow, the antibody to which the present invention relates differs from hitherto known antibodies in that it is capable of inhibiting the proliferation of tumour cells.

As has been stated hereinbefore, the CD151 protein belongs to the tetraspanin family and, by virtue thereof, contains 2 extracellular domains EC1 (18 amino acids, sequence [40-57]) and EC2 (109 amino acids, sequence [113-221]), also referred to as extracellular loops.

According to the present invention, the antibodies used are capable of binding to at least one epitope located in the extracellular domain. Preferably, said antibody will fix itself to the loops EC 1 and/or EC2.

More particularly, in accordance with a preferred embodiment of the invention, there is described the use of at least one anti-CD151 antibody, or one of its functional fragments, capable of binding to an epitope included in the extracellular loop 1 (EC1) and/or 2 (EC2), preferably EC2, corresponding to the amino acids 40-57 and 113-221, respectively, of the CD 151 protein.

The EC1 loop [40-57] contains 18 amino acids and has a theoretical weight of 2002.2 Da.

The EC2 loop [113-221] has an N-glycosylation site (residue Asn159) and 6 cysteine residues forming 3 disulfide bridges. A structural model of the EC2 loop of the tetraspanins, and especially of CD151, has been proposed on the basis of the three-dimensional structure of the EC2 loop of the tetraspanin CD81 (Seigneuret et al., 2001, J. Biol. Chem. 276, 40055-40064). According to that model, the tetraspanins have a common, relatively conserved scaffold composed of 3 α helices and a specific variable domain. For CD151, that scaffold is thought to be composed of the regions [113-157] and [209-221], and the variable domain is thought to be composed of the region [158-208].

The variable domain of the EC2 loop is thought to be more especially involved in the specific interactions of CD151 with proteins of the integrin family. Directed mutagenesis experiments have especially shown the importance of the region [193-208], and more precisely of the tripeptide QRD [194-196] and the cysteine residue at position 192, in the association of CD151 with certain laminin receptor integrins such as integrins α3β1 or α6β4 (Kazarov et al., 2002, J. Cell Biol. 158, 1299-1309).

Even more preferably, the present invention is directed at the use of at least one anti-CD151 antibody, or one of its functional fragments, capable of binding to an epitope of the EC2 region.

In a new aspect, the present invention relates to an isolated nucleic acid, **characterised in that** it is selected from the following nucleic acids:
a) a DNA or RNA nucleic acid coding for an antibody, or one of its derivative compounds or functional fragments, as defined hereinbefore;
b) a nucleic acid complementary to a nucleic acid as defined hereinbefore under a);
c) a nucleic acid of at least 18 nucleotides capable of hybridising under conditions of high stringency with at least one of the nucleic acid sequences SEQ ID Nos. 16-18 or 34-36 or with a sequence having at least 80%, preferably 85%, 90%, 95% and 98%, identity, after optimal alignment, with said sequences.

Are more preferred nucleic acid of at least 18 nucleotides capable of hybridising under conditions of high stringency with at least one of the nucleic acid sequences selected from the group of the following sequences:
- the sequence originated from the sequence SEQ ID No. 16 and encoding the fragment aa112-218 of the sequence SEQ ID No. 7;
- the sequence originated from the sequence SEQ ID No. 17 and encoding the fragment aa119-448 of the sequence SEQ ID No. 8;
- the sequence originated from the sequence SEQ ID No. 18 and encoding the fragment aa119-445 of the sequence SEQ ID No. 9;
- the sequence originated from the sequence SEQ ID No. 34 and encoding the fragment aa108-214 of the sequence SEQ ID No. 25;
- the sequence originated from the sequence SEQ ID No. 35 and encoding the fragment aa120-449 of the sequence SEQ ID No. 26; and
- the sequence originated from the sequence SEQ ID No. 36 and encoding the fragment aa120-446 of the sequence SEQ ID No. 27.

Table 3 hereinbelow summarises the different nucleotide sequences relating to antibodies according to the invention, with the CDR sequences defined according to IMGT and the chimeric light and heavy chains.

**Table 3**

| | c203B6 | c214B2 |
|---|---|---|
| CDR-L1 | 10 | 28 |
| CDR-L2 | 11 | 29 |
| CDR-L3 | 12 | 30 |
| Chim. VL | 16 | 34 |
| CDR-H1 | 13 | 31 |
| CDR-H2 | 14 | 32 |
| CDR-H3 | 15 | 33 |
| IgG1 Chim. VH | 17 | 35 |
| IgG4 Chim. VH | 18 | 36 |

The terms nucleic acid, nucleic or nucleic acid sequence, polynucleotide, oligonucleotide, polynucleotide sequence, nucleotide sequence, which will be used interchangeably in the present description, are understood as referring to a precise concatenation of nucleotides, modified or not, making it possible to define a fragment or region of a nucleic acid, including or not including non-natural nucleotides, which may correspond equally to a double-stranded DNA, a single-stranded DNA and to transcription products of said DNAs.

It must also be understood herein that the present invention does not relate to the nucleotide sequences in their natural chromosomic environment, that is to say in the natural state. They are sequences which have been isolated and/or purified, that is to say they have been extracted directly or indirectly, for example by copying, their environment having been at least partially modified. They are also understood herein to refer to isolated nucleic acids obtained by genetic recombination using, for example, host cells or obtained by chemical synthesis.

Nucleic sequences having a percentage identity of least 80%, preferably 85%, 90%, 95% and 98%, after optimal alignment, with a preferred sequence are understood to denote the nucleic sequences having, with respect to the reference nucleic sequence, certain modifications such as, especially, a deletion, truncation, extension, chimeric fusion and/or substitution, especially pointwise substitution. They are preferably sequences whose sequences code for the same amino acid sequences as the reference sequence, this being due to the degeneracy of the genetic code, or complementary sequences which are capable of hybridising specifically with the reference sequences, preferably under conditions of high stringency, especially as defined hereinbelow.

Hybridisation under conditions of high stringency means that the conditions for temperature and ionic strength are so selected that they make it possible for hybridisation to be maintained between two complementary fragments of DNA. By way of illustration, conditions of high stringency of the hybridisation step for the purpose of defining the polynucleotide fragments described hereinbefore are advantageously as follows.

DNA-DNA or DNA-RNA hybridisation is carried out in two steps: (1) prehybridisation at 42°C for 3 hours in phosphate buffer (20mM, pH 7.5) containing 5 x SSC (1 x SSC corresponds to a solution of 0.15M NaCl + 0.015M sodium citrate), 50% formamide, 7% sodium dodecyl sulfate (SDS), 10 x Denhardt's, 5% dextran sulfate and 1% salmon sperm DNA; (2) hybridisation proper for 20 hours at a temperature depending on the size of the probe (i.e.: 42°C for a probe of size > 100 nucleotides) followed by 2 washings of 20 minutes at 20°C in 2 x SSC + 2% SDS, 1 washing of 20 minutes at 20°C in 0.1 x SSC + 0.1% SDS. The final washing is carried out in 0.1 x SSC + 0.1% SDS for 30 minutes at 60°C for a probe of size > 100 nucleotides. The high-stringency conditions described hereinbefore for a polynucleotide of defined size can be adapted by the person skilled in the art for oligonucleotides of larger or smaller size, in accordance with the teaching of Sambrook et al., (1989, Molecular cloning: a laboratory manual. 2nd Ed. Cold Spring Harbor).

The invention relates also to a vector comprising a nucleic acid according to the present invention.

The invention is directed especially at cloning and/or expression vectors containing a nucleotide sequence according to the invention.

The vectors according to the invention preferably comprise elements which allow the expression and/or secretion of the nucleotide sequences in a particular host cell. The vector must then comprise a promoter, translation initiation and termination signals and also appropriate transcription regulation regions. It must be capable of being maintained in stable manner in the host cell and it may optionally have particular signals specifying the secretion of the translated protein. These various elements will be selected and optimised by the person skilled in the art as a function of the cell host used. To that effect, the nucleotide sequences according to the invention can be inserted into self replicating vectors within the selected host or can be integrative vectors of the selected host.

Such vectors are prepared by methods customarily used by the person skilled in the art, and the resulting clones can be introduced into a suitable host by standard methods such as lipofection, electroporation, heat shock or chemical methods.

The vectors according to the invention are, for example, vectors of plasmid or viral origin. They are useful in transforming host cells in order to clone or express the nucleotide sequences according to the invention.

The invention also includes the host cells transformed by or containing a vector according to the invention.

The cell host may be selected from prokaryotic or eukaryotic systems, for example, bacterial cells, but also yeast cells or animal cells, especially mammalian cells. Insect cells or plant cells can also be used.

The invention relates also to animals, with the exception of human beings, comprising a transformed cell according to the invention.

In accordance with another aspect, the invention relates to a method of producing an antibody, or one of its functional fragments, according to the invention, **characterised in that** it comprises the following steps:
a) culture of a host cell according to the invention in a suitable culture medium and under suitable culture conditions; and
b) recovery of said antibodies, or a functional fragment thereof, thereby produced, from the culture medium or from said cultured cells.

The transformed cells according to the invention can be used in methods for the preparation of recombinant polypeptides according to the invention. The methods for the preparation of a polypeptide according to the invention in recombinant form, **characterised in that** they employ a vector and/or a cell transformed by a vector according to the invention, are themselves included in the present invention. Preferably, a cell transformed by a vector according to the invention is cultured under conditions enabling the expression of said polypeptide, and said recombinant peptide is recovered.

As has been stated, the cell host may be selected from prokaryotic or eukaryotic systems. In particular, it is possible to identify nucleotide sequences according to the invention which facilitate secretion in such a prokaryotic or eukaryotic system. A vector according to the invention carrying such a sequence can therefore be advantageously used in the production of recombinant proteins that are intended to be secreted. Indeed, purification of these recombinant proteins of interest will be facilitated by the fact that they are present in the supernatant of the cell culture rather than in the interior of the host cells.

It is also possible to prepare the polypeptides according to the invention by chemical synthesis. Such a preparation method is also included in the subject-matter of the invention. The person skilled in the art knows methods of chemical synthesis, for example techniques employing solid phases (see, especially, Steward et al., 1984, Solid phase peptides synthesis, Pierce Chem. Company, Rockford, 111, 2nd Ed., (1984)) or techniques using partial solid phases, by means of condensation of fragments or by conventional synthesis in solution. The polypeptides obtained by chemical synthesis, which may contain corresponding non-natural amino acids, are also included in the invention.

The antibodies, or one of their derivative compounds or functional fragments, capable of being obtained by a method according to the invention are also included in the present invention.

According to yet another aspect, the present invention relates to an antibody as described hereinbefore, **characterised in that** it is, additionally, bispecific, that is to say capable of binding specifically to a human protein or human receptor other than CD151.

The bispecific, or bifunctional, antibodies constitute a second generation of monoclonal antibodies in which two different variable regions are combined in the same molecule (Hollinger and Bohlen, 1999, Cancer and metastasis rev. 18: 411-419). Their usefulness has been demonstrated both in the diagnostic field and in the therapeutic field by their ability to recruit new effector functions or to target a plurality of molecules on the surface of tumour cells. These antibodies can be obtained by chemical methods (Glennie MJ et al. 1987 J. Immunol. 139, 2367-2375; Repp R. et al. 1995 J. Hemat. 377-382) or somatic methods (Staerz U.D. and Bevan M.J. 1986 PNAS 83, 1453-1457; Suresh M.R. et al. 1986 Method Enzymol. 121: 210-228) but also, and preferably, by genetic engineering techniques which make it possible to force heterodimerisation and thereby facilitate the method of purifying the sought antibody (Merchand et al. 1998 Nature Biotech. 16: 677-681).

These bispecific antibodies can be constructed as whole IgGs, as bispecific Fab'2s, as Fab'PEGs or as diabodies or as bispecific scFvs but also as a tetravalent bispecific antibody in which two fixing sites are present for each antigen targeted (Park et al. 2000 Mol. Immunol. 37(18): 1123-30) or fragments thereof as described hereinbefore.

Besides an economic advantage due to the fact that production and administration of a bispecific antibody is less onerous than production of two specific antibodies, the use of such bispecific antibodies has the advantage of reducing the toxicity of the treatment. The use of a bispecific antibody makes it possible, in fact, to reduce the overall amount of circulating antibodies and, as a result, the possible toxicity.

In a preferred embodiment of the invention, the bispecific antibody is a divalent or tetravalent antibody.

Finally, the present invention is directed to the antibody, or one of its derivative compounds or functional fragments, as described hereinbefore, as a medicament.

The invention relates also to a pharmaceutical composition comprising, as active ingredient, a compound consisting of an antibody, or one of its derivative compounds or functional fragments, according to the invention. Preferably, there is added to said antibody an excipient and/or a pharmaceutically acceptable carrier.

According to yet another embodiment, the present invention relates also to a pharmaceutical composition as described hereinbefore which additionally comprises, as a combination product for simultaneous, separate or time-staggered use, at least one other antibody, a cytotoxic/cytostatic agent, a cell toxin or a radioelement.

"Simultaneous use" is understood as the administration of the two compounds of the composition according to the invention contained in one and the same pharmaceutical form.

"Separate use" is understood as the administration, at the same time, of the two compounds of the composition according to the invention contained in separate pharmaceutical forms.

"Time-staggered use" is understood as the successive administration of the two compounds of the composition according to the invention, each contained in a separate pharmaceutical form.

In general manner, the composition according to the invention considerably increases the efficacy of the cancer treatment. In other words, the therapeutic effect of the antibody according to the invention is potentiated in unexpected manner by the administration of a cytotoxic agent. Another major subsequent advantage produced by a composition according to the invention relates to the possibility of using lower effective doses of active ingredient, which makes it possible to avoid or reduce the risks of secondary effects appearing, especially the effect of the cytotoxic agent. Moreover, this composition according to the invention should make it possible to achieve the expected therapeutic effect more rapidly.

"Anti-cancer therapeutic agents" or "cytotoxic agents" should be understood as substances which, when administered to a patient, treats or prevents the development of the cancer in the patient. By way of non-limiting example of such agents there may be mentioned "alkylating" agents, antimetabolites, anti-tumour antibiotics, mitotic inhibitors, chromatin function inhibitors, anti-angiogenesis agents, anti-oestrogens, anti-androgens or immunomodulators.

Such agents are, for example, mentioned in the VIDAL, on the page devoted to oncology and haematology in the column "Cytotoxiques" (English: cytotoxic agents); such cytotoxic compounds mentioned by way of reference to that document are mentioned here as preferred cytotoxic agents.

"Alkylating agents" refer to any substance which is capable of covalently binding to or alkylating any molecule, preferably a nucleic acid (e.g.: DNA), within a cell. As examples of such alkylating agents there may be mentioned nitrogen mustards such as mechlorethamine, chlorambucil, melphalan hydrochloride, pipobroman, prednimustine disodium phosphate or estramustine; oxazophorines such as cyclophosphamide, altretamine, trofosfamide, sulfofosfamide or ifosfamide; aziridines or ethylene-imines such as thiotepa, triethyleneamine or altetramine; nitrosoureas such as carmustine, streptozocin, fotemustine or lomustine; alkyl sulfonates such as busulfan, treosulfan or improsulfan; triazenes such as dacarbazine; and also platinum complexes such as cisplatin, oxaliplatin or carboplatin.

"Antimetabolites" refer to substances which block cell growth and/or cell metabolism by interfering with certain activities, generally DNA synthesis. By way of example of antimetabolites there may be mentioned methotrexate, 5-fluorouracil, floxuridine, 5-fluorodeoxyuridine, capecitabine, cytarabine, fludarabine, cytosine arabinoside, 6-mercaptopurine (6-MP), 6-thioguanine (6-TG), chlorodeoxyadenosine, 5-azacytidine, gemcitabine, cladribine, deoxycoformycin and pentostatin.

"Anti-tumour antibiotics" refer to compounds which can prevent or inhibit the synthesis of DNA, of RNA and/or of proteins. Examples of such anti-tumour antibiotics include doxorubicin, daunorubicin, idarubicin, valrubicin, mitoxantrone, dactinomycin, mithramycin, plicamycin, mitomycin C, bleomycin and procarbazine.

"Mitotic inhibitors" prevent the normal progression of the cell cycle and mitosis. In general, the microtubule inhibitors or "taxoids" such as paclitaxel and docetaxel are capable of inhibiting mitosis. The vinca alkaloids such as vinblastine, vincristine, vindesine and vinorelbine are also capable of inhibiting mitosis.

"Chromatin function inhibitors" or "topoisomerase inhibitors" refer to substances which inhibit the normal function of chromatin remodelling proteins such as topoisomerases I and II. Examples of such inhibitors include, for topoisomerase I, camptothecin and also its derivatives such as irinotecan or topotecan and, for topoisomerase II, etoposide, etiposide phosphate and teniposide.

"Anti-angiogenesis agents" refer to any drug, compound, substance or agent which inhibits the growth of blood vessels. Examples of anti-angiogenesis agents include, without any limitation, razoxin, marimastat, batimastat, prinomastat, tanomastat, ilomastat, CGS-27023A, halofuginone, COL-3, neovastat, BMS-275291, thalidomide, CDC 501, DMXAA, L-651582, squalamine, endostatin, SU5416, SU6668, interferon-alpha, EMD121974, interleukin-12, IM862, angiostatin and vitaxin.

"Anti-oestrogens" or "anti-oestrogen agents" refer to any substance which reduces, antagonises or inhibits the action of oestrogens. Examples of such agents are tamoxifen, toremifene, raloxifene, droloxifene, iodoxyfene, anastrozole, letrozole and exemestane.

"Anti-androgens" or "anti-androgen agents" refer to any substance which reduces, antagonises or inhibits the action of an androgen. Examples of anti-androgens are flutamide, nilutamide, bicalutamide, spironolactone, cyproterone acetate, finasteride and cimitidine.

Immunomodulators are substances which stimulate the immune system. Examples of such immunomodulators include interferons, interleukins such as aldesleukin, OCT-43, denileukin diflitox or interleukin-2, tumour necrosis factors such as tasonermin, or other types of immunomodulators such as lentinan, sizofiran, roquinimex, pidotimod, pegademase, thymopentin, poly I:C, or levamisole in combination with 5-fluorouracil.

For further details, the person skilled in the art will be able to refer to the manual published by the French Association of Teachers of Therapeutic Chemistry entitled "Traité de chimie thérapeutique, Vol. 6, Médicaments antitumoraux et perspectives dans le traitement des cancers, ed. TEC & DOC, 2003".

In an especially preferred embodiment, said composition in the form of a combination product according to the invention is **characterised in that** said cytotoxic agent is chemically bound to said antibody for simultaneous use.

In an especially preferred embodiment, said composition according to the invention is **characterised in that** said cytotoxic/cytostatic agent is selected from spindle inhibitor or stabiliser agents, preferably vinorelbine and/or vinflunine and/or vincristine.

In order to facilitate binding between said cytotoxic agent and said antibody according to the invention, it will be possible, especially, to introduce spacer molecules between the two compounds to be bound, e.g. poly(alkylene)glycols such as polyethyleneglycol, or also amino acids, or, in another embodiment, to use active derivatives of said cytotoxic agents into which there will have been introduced functions capable of reacting with said antibody according to the invention. These binding techniques are will known to the person skilled in the art and will not be elaborated upon in the present description.

According to another aspect, the invention relates to a composition **characterised in that** one, at least, of said antibodies, or one of their derivative compounds or functional fragments, is conjugated with a cell toxin and/or a radioelement.

Preferably, said toxin or said radioelement is capable of preventing the growth or proliferation of the tumour cell, especially of totally inactivating said tumour cell.

Preference is also given to said toxin being an enterobacterial toxin, especially Pseudomonas exotoxin A.

The radioelements (or radioisotopes) employed in therapy, preferably conjugated with the antibody, are radioisotopes which emit gamma rays, preferably iodine¹³¹, yttrium⁹⁰, gold¹⁹⁹, palladium¹⁰⁰, copper⁶⁷, bismuth²¹⁷ and antimony²¹¹. Radioisotopes which emit beta and alpha rays may also be used in therapy.

A toxin or radioelement conjugated with at least one antibody, or a functional fragment thereof, according to the invention is understood to refer to any means making it possible to bind said toxin or said radioelement to said at least one antibody, especially by covalent binding between the two compounds, with or without introduction of a linking molecule.

Among the agents allowing chemical (covalent), electrostatic or non-covalent linkage of all or some of the conjugate's elements there may be mentioned, very especially, benzoquinone, carbodiimide and, more especially, EDC (1-ethyl-3-[3-dimethylaminopropyl]-carbodiimide hydrochloride), dimaleimide, dithiobis-nitro-benzoic acid (DTNB), N-succinimidyl S-acetyl thioacetate (SATA), agents referred to as "bridging" agents having one or more groups, with one or more phenylazide groups, reacting with ultraviolet (UV) and very preferably N-[-4-(azidosalicylamino)butyl]-3'-(2'-pyridyldithio)propionamide (APDP), N-succinimidyl 3-(2-pyridyldithio)propionate (SPDP) and 6-hydrazino-nicotinamide (HYNIC).

Another form of binding, very especially for radioelements, can consist of using a bifunctional ion chelator.

Among those chelators there may be mentioned the chelators derived from EDTA (ethylenediaminetetraacetic acid) or DTPA (diethylenetriaminepentaacetic acid) that have been developed for binding metals, especially radioactive metals, and immunoglobulins. Accordingly, DTPA and its derivatives can be substituted with different groups on the carbon chain so as to increase the stability and rigidity of the ligand-metal complex (Krejcarek *et al.* (1977); Brechbiel *et al.* (1991); Gansow (1991); US patent 4,831,175).

For example, DTPA (diethylenetriaminepentaacetic acid) and its derivatives, which has long been used very widely in medicine and biology either in its free form or in the form of a complex with a metal ion, has the noteworthy characteristic of forming stable chelates with metal ions and of being bound to proteins of therapeutic or diagnostic interest such as antibodies for the development of radioimmunoconjugates in cancer therapy (Meases *et al.,* (1984); Gansow *et al.* (1990)).

Also preferably, said at least one antibody forming said conjugate according to the invention is selected from its functional fragments, especially fragments lacking the Fc component such as scFv fragments.

The present invention additionally comprises use of the composition according to the invention in the preparation of a medicament intended for the prevention or treatment of cancer.

The present invention relates also to the use of an antibody, or one of its derivative compounds or functional fragments, preferably humanised, and/or of a composition according to the invention in the preparation of a medicament intended to inhibit the proliferation of tumour cells. In general manner, the present invention relates to the use of an antibody, or one of its derivative compounds or functional fragments, preferably humanised, and/or of a composition according to the invention in the preparation of a medicament intended for the prevention or treatment of cancer.

Among the cancers that may be prevented and/or treated, preference is given to prostate cancer, osteosarcomas, lung cancer, breast cancer, endometrial cancer, colon cancer, multiple myeloma or ovarian cancer, pancreatic cancer or any other cancer.

Preferably, said cancer is a cancer selected from prostate cancer, lung cancer, colon cancer, breast cancer and/or pancreatic cancer.

In accordance with yet another aspect, the present invention relates to the use of the antibody according to the invention in a, preferably *in vitro,* diagnostic method for diseases associated with a level of expression of CD151. More especially, the invention is directed to an *in vitro* diagnostic method for diseases having overexpression or underexpression of the CD151 protein, starting from a biological sample in which the abnormal presence of the CD151 protein is suspected, said method consisting of placing said biological sample in contact with an antibody according to the invention, it being possible for said antibody, where appropriate, to be labelled.

Preferably, said diseases associated with the CD151 protein in said diagnostic method will be cancers.

Said antibody, or one of its functional fragments, can be in the form of an immunoconjugate or antibody labelled in order to obtain a detectable and/or quantifiable signal.

The antibodies labelled according to the invention or their functional fragments include, for example, antibodies referred to as immunoconjugates which can be conjugated, for example, with enzymes such as peroxidase, alkaline phosphatase, α-D-galactosidase, glucose oxidase, glucose amylase, carbonic anhydrase, acetylcholinesterase, lysozyme, malate dehydrogenase or glucose-6 phosphate dehydrogenase or with a molecule such as biotin, digoxigenin or 5-bromo-deoxyuridine. Fluorescent labels can also be conjugated with the antibodies, or their functional fragments, according to the invention and include, especially, fluorescein and its derivatives, fluorochrome, rhodamine and its derivatives, GFP (Green Fluorescent Protein), dansyl, umbelliferone etc.. In such conjugates, the antibodies of the invention or their functional fragments can be prepared by methods known to the person skilled in the art. They can be bound to the enzymes or fluorescent labels directly or by way of a spacer group or a linkage group such as a polyaldehyde, e.g. glutaraldehyde, ethylenediaminetetraacetic acid (EDTA) or diethylenetriaminepentaacetic acid (DPTA), or in the presence of binding agents such as those mentioned hereinbefore for the therapeutic conjugates. Conjugates comprising fluorescein-type labels can be prepared by reaction with an isothiocyanate.

Other conjugates can also include chemoluminescent labels such as luminol and dioxetanes, bioluminescent labels such as luciferase and luciferin, or also radioactive labels such as iodine¹²³, iodine¹²⁵, iodine¹²⁶, iodine¹³³, bromine⁷⁷, technetium^{99m}, indium¹¹¹, indium^{113m}, gallium⁶⁷, gallium⁶⁸, ruthenium⁹⁵, ruthenium⁹⁷, ruthenium¹⁰³, ruthenium¹⁰⁵, mercury¹⁰⁷, mercury²⁰³, rhenium^{99m}, rhenium¹⁰¹, rhenium¹⁰⁵, scandium⁴⁷, tellurium^{121m}, tellurium^{122m}, tellurium^{125m}, thulium¹⁶⁵, thulium¹⁶⁷, thulium¹⁶⁸, fluorine¹⁸, yttrium¹⁹⁹, iodine¹³¹. The methods known to the person skilled in the art that exist for binding radioisotopes to the antibodies, either directly or via a chelating agent such as EDTA or DTPA mentioned hereinbefore, can be used for the radioelements in diagnostics. There may also therefore be mentioned labelling with [I¹²⁵]Na by the chloramine T technique [Hunter W.M. and Greenwood F.C. (1962) Nature 194:495] or also with technetium^{99m} by the technique of Crockford et al. (US patent 4 424 200) or fixed via DTPA as described by Hnatowich (US patent 4 479 930).

The invention relates also to the use of an antibody according to the invention in the preparation of a medicament intended for the specific targeting of a biologically active compound at cells expressing or overexpressing the CD151 protein.

A biologically active compound is understood herein as referring to any compound capable of modifying, especially inhibiting, the activity of cells, especially their growth, their proliferation, or the transcription or translation of genes.

The invention relates also to an *in vivo* diagnostic reagent comprising an antibody according to the invention, or one of its functional fragments, preferably labelled, especially radiolabelled, and to its use in medical imaging, especially in the detection of cancer associated with the expression or overexpression of the CD151 protein by a cell.

The invention relates also to a composition in the form of a combination product or to an anti-CD151/toxin or radioelement conjugate, according to the invention, as a medicament.

Preferably, to said composition in the form of a combination product or to said conjugate according to the invention there will be added an excipient and/or a pharmaceutically acceptable carrier.

In the present description, a pharmaceutically acceptable carrier is understood as referring to a compound or combination of compounds included in a pharmaceutical composition which does not give rise to secondary reactions and which, for example, makes it possible to facilitate the administration of the active compound(s), to increase the life or efficacy thereof in the body, to increase the solubility thereof in solution or to improve its storage. Such pharmaceutically acceptable carriers are well-known and will be adapted by the person skilled in the art as a function of the nature and mode of administration of the selected active compound(s).

Preferably, those compounds will be administered by a systemic route, especially the intravenous route, by the intramuscular, intradermic, intraperitoneal or subcutaneous route, or by the oral route. More preferably, the composition comprising the antibodies according to the invention will be administered on a plurality of occasions staggered over time.

Their optimal modes of administration, dosage regimens and galenic forms can be determined according to criteria generally taken into consideration in establishing a suitable treatment for a patient such as, for example, the age or bodyweight of the patient, the severity of his or her general condition, the tolerability of the treatment and the secondary effects established.

The invention relates accordingly to the use of an antibody, or one of its functional fragments, in the preparation of a medicament intended for specifically targeting a biologically active compound at cells expressing or overexpressing CD151.

Other characteristics and advantages of the invention will emerge in the remainder of the description with the Examples and Figures, for which the legends are given hereinbelow.

### LEGENDS FOR FIGURES

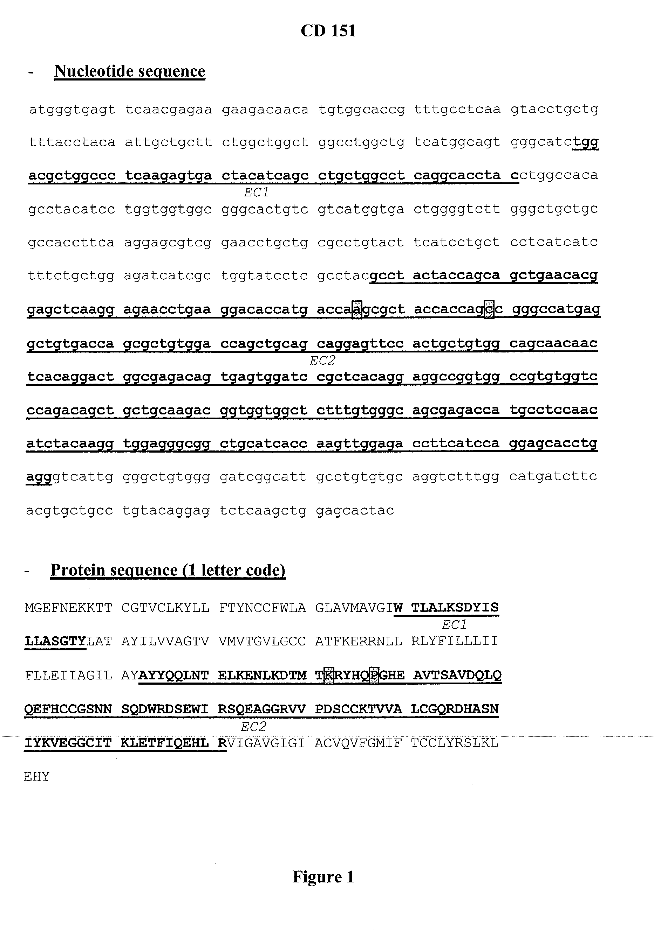

Figure 1 shows the nucleotide and protein sequences (respectively SEQ ID No.37 and SEQ ID No. 38) of the CD151 protein, on which sequences there are shown the EC 1 and EC2 loops.

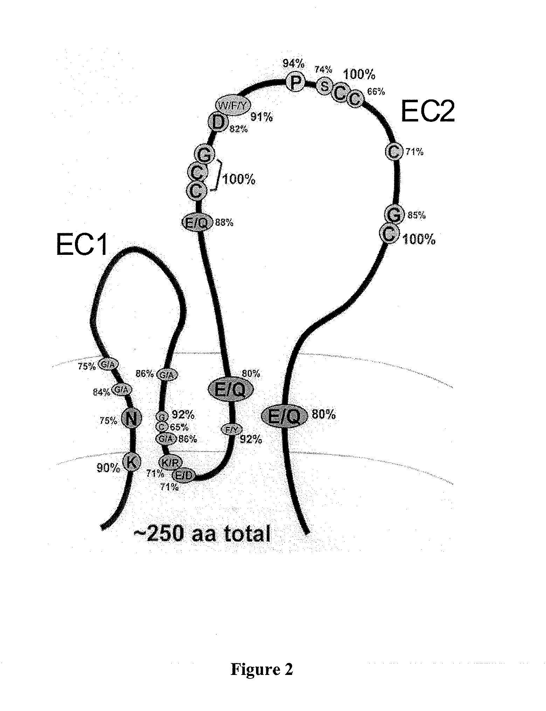

Figure 2 is a diagram illustrating the structure of the tetraspanins, to which the CD151 protein belongs, and very especially the two extracellular loops EC 1 and EC2.
Figures 3A-3E illustrate the expression of the CD151 molecule in patients suffering from prostate cancer. Each letter corresponds to study of one patient and for each patient the upper panel corresponds to the normal tissue adjacent to the tumour and the lower panel corresponds to the tumour tissue.
Figures 4A-4C illustrate the expression of the CD151 molecule in patients suffering from lung cancer. Each letter corresponds to study of one patient and for each patient the upper panel corresponds to the normal tissue adjacent to the tumour and the lower panel corresponds to the tumour tissue.
Figures 5A-5D show the analysis, by flow cytometry, of the recognition of CD151 by the murine antibody 203B6 on the surface of NIH 3T3-CD151, PC3 and A549 cells.
Figures 6A-6D show the analysis, by flow cytometry, of the recognition of CD151 by the murine antibody 214B2 on the surface of NIH 3T3-CD151, PC3 and A549 cells.
Figure 7 represents the alignment between mouse 214B2 V- and J- regions and closest mouse germline genes for heavy and light chains.
Figure 8 illustrates the humanization of 214B2 light chain with the different Priority Ranking: #1 is high priority (putative high impact on target recognition, CDR presentation and overall 3D structure) ; #2 is medium priority ; and #3 is low priority (putative low impact on target recognition, CDR presentation and overall 3D structure).
Figure 9 illustrates the humanization of 214B2 heavy chain with the different Priority Ranking: #1 is high priority (putative high impact on target recognition, CDR presentation and overall 3D structure); #2 is medium priority; and #3 is low priority (putative low impact on target recognition, CDR presentation and overall 3D structure).
Figure 10 illustrates the specificity study of chimeric antibodies c203B6[IgG1] and c214B2[IgG1] by western blot.
Figures 11A-11B illustrate the inhibition of binding of murine Mabs 203B6 and 214B2 to recombinant EC2 by their respective chimeric forms: c203B6[IgG1] (A); c214B2[IgG1] (B).
Figures 12A-12B: Binding of chimeric antibodies to PC3 cells with A: c214B2[IgG1] and B: c203B6[IgG1].
Figure 13 illustrates the *in vivo* activity of the c214B2[IgG1] Mab on tumor growth of PC3, an androgen-independent prostate cell line.

### EXAMPLES

### Example 1: Study of expression of the CD151 molecule

The expression of the CD 151 protein was researched by immunohistochemistry (IHC) in samples of human tissues obtained from patients suffering from prostate cancers or lung cancer. For these patients, slides of normal tissues adjacent to the tumour were available and were therefore included in order to calibrate the level of expression in the tumour tissues *versus* normal tissues.

For these experiments, commercially available slides of the "Tissue array" type are used. After deparaffinisation, antigen unmasking is performed at 30°C with the aid of an enzymatic solution containing pepsin (Labvision ref. AP-9007-005). This step is followed by a step of removal of endogenous peroxidases by incubation of the sections in a solution of hydrogen peroxide (Sigma) 0.3% in water. Saturation of the non-specific sites is then carried out with a solution of Ultra-V-Block (Labvision, ref. TA-125-UB) and labelling is carried out using a commercially available murine anti-CD151 antibody (Serotech, Ref. MCA 1856) used at a final concentration of 5 µg/ml. A murine IgG1 isotype control antibody (DakoCytomation, Ref. X0931) is used as a negative experimental control. Labelling visualisation is performed using the Envision Dual Link visualisation system (DakoCytomation, Ref. K4061) and the reference of the DAB peroxidase substrate is S3309 from DakoCytomation.

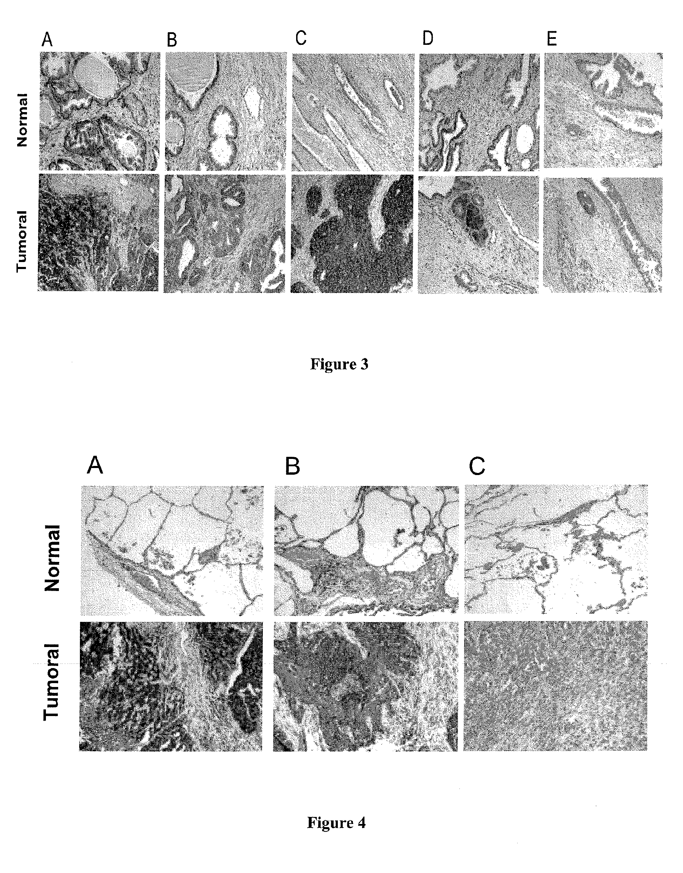

The results presented in Figures 3A-3E show that a number of patients developing prostate tumours exhibit overexpression of the CD151 molecule. This overexpression may be very significant for 20% of the patients studied (patients A and C) or moderate (patients A and D). It is to be noted that, except at the level of the endothelial cells, the corresponding normal prostatic tissues do not express CD151 or express it only a little and that, where it is expressed, it seems to be limited to glandular type structures. Patient E exhibits an example of a tumour not expressing CD151.

In the case of the lung cancer (Figures 4A-4C), moderate (patient A) to marked (patient B) expression is observed in certain cells of normal pulmonary tissue. However, the tumour tissue exhibits a very high density of heavily labelled cells (patients A and B). Patient C exhibits an example of a tumour not expressing CD151.

### Example 2: Generation and selection of the antibodies

BALB/c mice were immunised by the subcutaneous route using 20x10⁶ NIH 3T3 cells expressing human CD151 on their surface, those cells having been generated by transfection with the CD 151 gene. The first immunisation was carried out in the presence of Freund's complete adjuvant, and the next 2 in the presence of Freund's incomplete adjuvant. Three days before fusion, a final booster injection of 10x10⁶ NIH 3T3-CD151 cells was carried out by the intraperitoneal route. Mouse spleen cells were then fused to SP2/0-Ag14 myeloma cells in a ratio of 1/1 using conventional techniques described by Köhler and Milstein.

The antibodies secreted into the supernatants from the hybridomas resulting from the fusion were then screened for their ability to recognise the recombinant extracellular loop EC2 of CD151 by ELISA, and CD151 expressed on the surface of the human PC3 prostate cancer tumour line by flow cytometry.

For the ELISA, 96-well plates are sensitised for 1 night at +4°C with the recombinant extracellular loop EC2 at 5 µg/ml in PBS. After washing with PBS, the wells are saturated with 0.5% gelatin solution in PBS for 1 hour at 37°C and then washed again with PBS. The hybridoma culture supernatants are evaluated without dilution (incubation for 1 hour at 37°C). The antibodies fixed to the immobilised EC2 loop are detected by successive incubation with a peroxidase-conjugated goat anti-mouse IgG polyclonal antibody (Jackson/USA, dilution to 1/5000, 1 hour at 37°C) and then with a peroxidase substrate (TMB, Interchim/France, 10 minutes at ambient temperature). The reaction is stopped by addition of 1M sulfuric acid and the optical density (OD) is measured at 450 nm.

The flow cytometry analyses are carried out on 96-well plates. The undiluted hybridoma supernatants are added to 100000 PC3 cells previously introduced into the wells. After incubation for 20 minutes at +4°C followed by washing, an Alexa488-labelled goat anti-mouse IgG polyclonal antibody (Molecular Probes, dilution to 1/500) is added. The fluorescence intensity (MFI) is determined with the aid of a cytofluorimeter after further incubation for 20 minutes at +4°C.

At the end of that screening, the following 2 hybridomas were selected (selection criteria: OD > 0.5 for the ELISA and MFI > 50 for the flow cytometry): 203B6, and 214B2. The results obtained for those 2 hybridomas are presented in Table 4 below:

**Table 4**

| Hybridoma | ELISA OD 450nm | Cytometry (PC3) MFI |
|---|---|---|
| 203B6 | 1.130 | 975 |
| 214B2 | 0.684 | 1004 |

After cloning, a clone of each selected hybridoma was amplified. The isotypes of the antibodies produced were determined for each culture supernatant using a murine antibody isotyping kit (SBA clonotyping system, Southern Biotech), and then final characterisation was carried out by ELISA (extracellular loop EC2) and by flow cytometry on the murine line NIH 3T3 and the stable transfectant NIH 3T3-CD151, and then on human tumour lines of lung cancer A549, prostate cancer DU145 and pancreatic cancer BxPC3 under the conditions previously described. The antibody concentration of the supernatants was adjusted to 5 µg/ml for the ELISA and to 10 µg/ml for the flow cytometry analyses. The results obtained are presented in Table 5 below:

**Table 5**

| Anti-CD151 antibody | Isotype | ELISA OD at 450nm | Flow cytometry MFI | | | | |
|---|---|---|---|---|---|---|---|
| | | | NIH3T3 | NIH3T3-CD151 | A549 | DU145 | BxPC3 |
| 203B6 cl1A | IgG1 K | 2.218 | 16 | 540 | 675 | 860 | 768 |
| 214B2 cl1A | IgG1 K | 2.477 | 16 | 748 | 820 | 1332 | 849 |

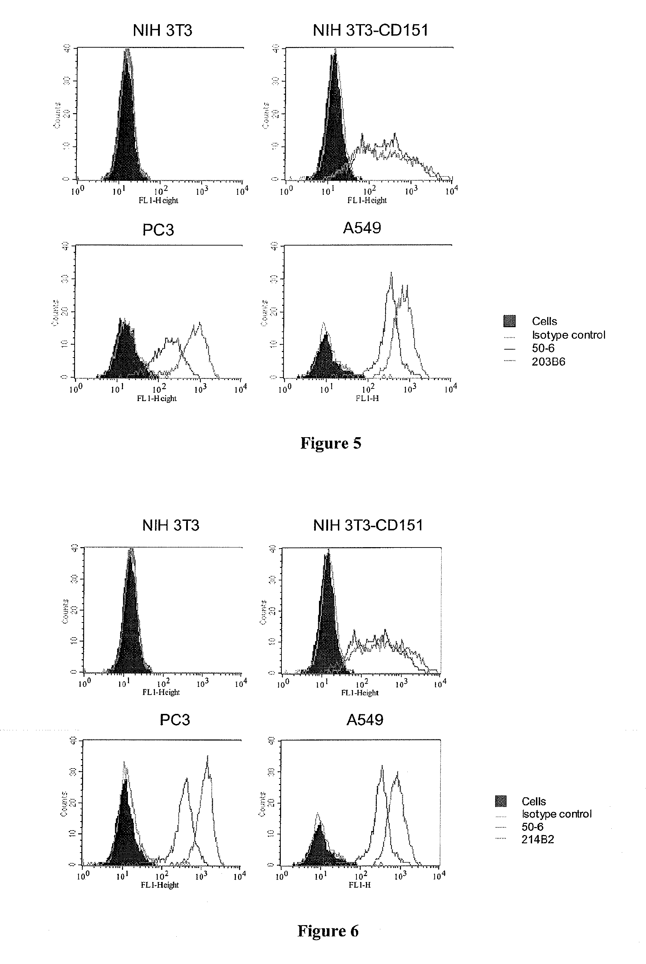

Figures 5A-5D and 6A-6D show the recognition profiles for the NIH 3T3-CD151, PC3 and A549 cells, by flow cytometry, which are obtained for the murine antibodies 203B6, and 214B2. These profiles are comparable to those obtained with the anti-CD151 antibody 50-6 (ATCC CRL-2696) and demonstrate the specificity of these antibodies for CD 151.

The hybridomas were then deposited at the CNCM, Collection Nationale de Cultures de Microorganismes, Institut Pasteur, 25 Rue du Docteur Roux, 75724 PARIS Cedex 15.

### Example 3: Cloning and production of chimeric and humanized antibodies

Chimeric formats of murine 203B6 and 214B2 Mab were designed: they correspond to the light and heavy chain variable domains of the murine antibodies of interest, genetically fused to human Ckappa and either IgG1 and IgG4 constant domains. Similarly, the below described humanized forms are produced as human IgG1/kappa or IgG4/kappa molecules. All recombinant Mabs are produced upon transient transfection by using the HEK293/EBNA system with a pCEP4 expression vector (InVitrogen, US).

The entire nucleotide sequences corresponding to the variable domains of 203B6 and 214B2 Mab light and heavy chains (chimeric or humanized) were synthesized by global gene synthesis (Genecust, Luxembourg). They were subcloned into a pCEP4 vector (InVitrogen, US) carrying the entire coding sequence of the constant domain of either the light [Ckappa] or the heavy [CH1-Hinge-CH2-CH3] chain of a human IgG1 or human IgG4 immunoglobulin. All cloning steps were performed according to conventional molecular biology techniques as described in the Laboratory manual (Sambrook and Russel, 2001) or according to the supplier's instructions. Each genetic construct was fully validated by nucleotide sequencing using Big Dye terminator cycle sequencing kit (Applied Biosystems, US) and analyzed using a 3100 Genetic Analyzer (Applied Biosystems, US).

Suspension-adapted HEK293 EBNA cells (InVitrogen, US) were routinely grown in 250 ml flasks in 50 ml of serum-free medium Excell 293 (SAFC Biosciences) supplemented with 6 mM glutamine on an orbital shaker (110 rpm rotation speed). Transient transfection was performed with 2.10⁶ cells/ml using linear 25 kDa polyethyleneimine (PEI) (Polysciences) prepared in water at a final concentration of 1 mg/ml mixed and plasmid DNA (final concentration of 1.25 µg/ml for heavy to light chain plasmid ratio of 1:1). At 4 hours post-transfection, the culture was diluted with one volume of fresh culture medium to achieve a final cell density of 10⁶ cells/ml. Cultivation process was monitored on the basis of cell viability and Mab production. Typically, cultures were maintained for 4 to 5 days. Mabs were purified using a conventional chromatography approach on a Protein A resin (GE Healthcare, US).

All different Mabs are produced at levels suitable with functional evaluations.

### Example 4: Humanization of mouse anti-CD151 antibody 214B2 variable domains

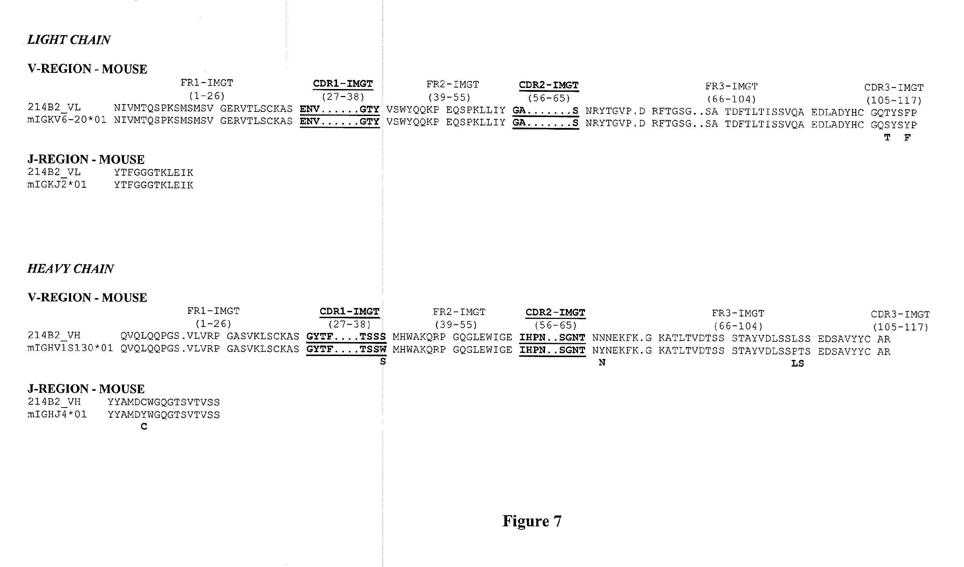

The heavy and light chain variable domains VH and VL from mouse 214B2 antibody were submitted to an immunogenetic analysis by using the IMGT libraries and tools (http://imgt.cines.fr). The below described humanization strategy is based on the unique IMGT numbering scheme (Lefranc, 1997). First, the selected mouse germlines for each domain was identified via a BLAST search against IMGT LIGM DB database, by using the DomainGapAlign tool. The mouse IGKV6-20*01 germline yielded 97.9 % identity with the rearranged 214B2 V-region and IGKJ2*01 was fully identical to the 214B2 J-region (Figure 7). Concerning the heavy chain, 214B2 V-region is most homologous to the mouse IGHVS130*01 germline (about 96 % identity, Fig. 7) and concerning the J-region, mouse IGHJ4*01 corresponds to the closest J germline gene (94 % identity, one divergent residue, Fig 7). The Diversity D-germline gene corresponds to mouse IGHD5-1*01, it corresponds essentially to CDR3.

As an example of mouse 214B2 Mab humanization, a conventional strategy by CDR-grafting is described below. Each heavy and light chain is humanized individually and evaluated by co-expression either with its respective chimeric or humanized counterpart.

### - Humanization of 214B2 light chain VL

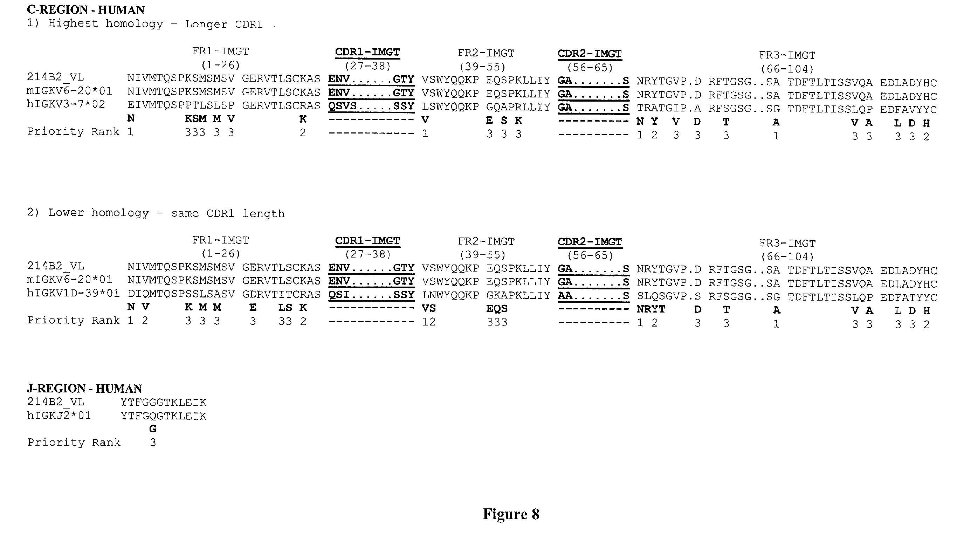

An atypical residue corresponding to Asn1 is identified, it exists in some mouse germline V-genes but never in human. Since it is located in proximity to CDR1, its modification into Glu as found in the selected human V-genes may me cautious and must be evaluated (Figure 8). Otherwise, search for the most suitable human germline for grafting mouse 214B2 light chain CDRs identified two potential hits. The first corresponds to the human V-region showing the closest degree of homology with mouse IGKV6-20*01, it is human IGKV3-7*02 germline allele (68.4 % identity). Nevertheless, its CDR1 is one amino acid longer than the one of 214B2, thus these CDR anchors are eventually not best suited for CDR1 grafting (see Fig 8). A second human donor germline can be IGKV1D-39*01; it is 64 % identical to mouse IGKV6-20*01 but CDR lengths are identical (6:3, Fig. 8). Criteria to evaluate the putative importance of each of the divergent residues between 214B2 VL and selected human V-gene include but are not restricted to: localization in the Vernier zone, location close to CDR anchors, presence at same position in another allele of same human germline group.

Considering IGKV6-20*01 V-gene, one residue within the Vernier zone is different as compared to both m214B2 and mIGKV6-20*02 (A84), it must be considered with high priority and conserved as mouse in a first instance (#1, Fig. 8). Otherwise, two important residues correspond to the CDR1 and CDR2 anchors V39 and N66, they are as well ranked with high priority and conserved as mouse in a first instance. Three residues are ranked as medium priority residues to consider because of their position close to CDR anchors (K24, Y68 and H103, Fig. 8). The remaining amino acids (ranked as #3) are likely to have a weak impact on the overall conformation of the humanized heavy chain and on CD151 recognition, they can easily be replaced by their human counterpart.

Considering IGKV1D-39*01 V-gene, one residue within the Vernier zone is different as compared to both m214B2 and mIGKV6-20*02 (A84), it must be considered with high priority and conserved as mouse in a first instance (#1, Fig. 8). Otherwise, two important residues correspond to the CDR1 and CDR2 anchors V39 and N66, they are as well ranked with high priority and conserved as mouse in a first instance. Five residues are ranked as medium priority residues to consider because of their position close to CDR anchors (K24, S40, Y68 and H103; Fig. 8) or in proximity to CDR1 (V3, Fig. 8). The remaining amino acids (ranked as #3) are likely to have a weak impact on the overall conformation of the humanized heavy chain and on CD151 recognition, they can easily be replaced by their human counterpart.

Considering the J-region, the human IGKJ2*01 gene contains 1 divergent residue (G) as compared to both m214B2 and mIGKJ2*01. Since this region corresponds essentially to FR4, it will be fully humanized in a first instance (Fig. 8).

The IMGT-CDR3 sequence (GQTYSFPYT) will be grafted per se without sequence modification.

### - Humanization of 214B2 heavy chain

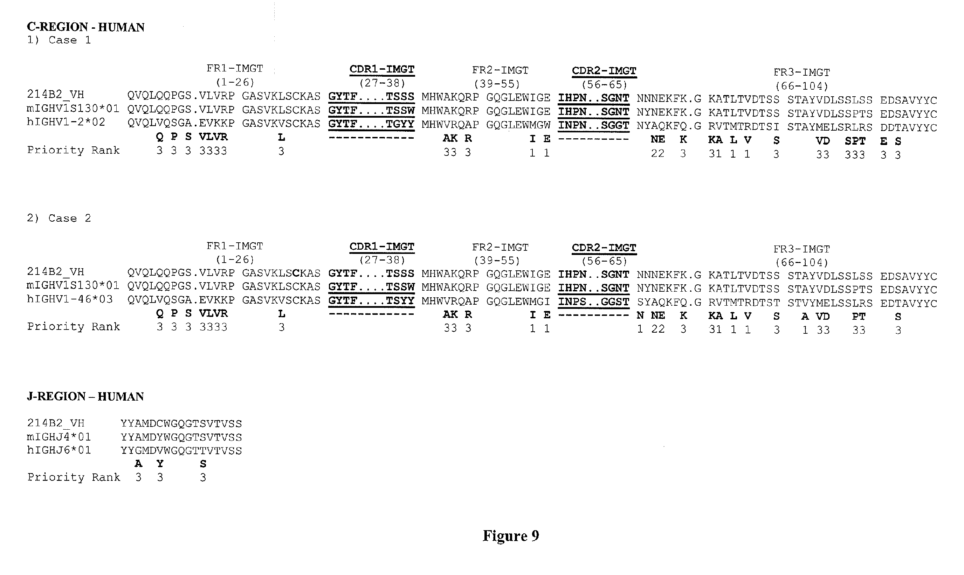

Search for the most suitable human germline for grafting mouse 214B2 heavy chain CDRs identified one preferential hit corresponding to human IGHV1-2*02 V-gene allele. It is 66 % identical to the mouse IGHVS130*01 germline gene, and CDR lengths are identical. Twenty-eight positions are divergent between 214B2_VH and IGHV1-2*02 in their FRs (Figure 9). The closely related human IGHV1-46*03 germline may as well be suitable, it yielded the same amount of divergent residues, and similarly 15 out of 28 are located in FR3. Criteria to evaluate the putative importance of each of these 28 divergent residues include but are not restricted to: localization in the Vernier zone, location close to CDR anchors, presence at same position in another allele of same human germline group.

Considering IGHV1-2*02 V-gene, five residues within the Vernier zone are different as compared to both m214B2 and mIGHVS130*01 (153, E55, A76, L78, V80), they must be considered with high priority and conserved as mouse in a first instance (#1, Fig. 9). Otherwise, N68 and E69 are ranked as medium priority residues to consider because of their position close to the CDR2 anchor (Fig. 9). The 21 remaining amino acids are likely to have a weak impact on the overall conformation of the humanized heavy chain and on CD151 recognition, they can easily be replaced by their human counterpart.

Considering IGHV1-46*03 V-gene, seven residues within the Vernier zone are different as compared to both m214B2 and mIGHVS130*01 (153, E55, N66, A76, L78, V80, A87), they must be considered with high priority and conserved as mouse in a first instance (#1, Fig. 9). Otherwise, N68 and E69 are ranked as medium priority residues to consider because of their position close to the CDR2 anchor (Fig. 9). The 19 remaining amino acids are likely to have a weak impact on the overall conformation of the humanized heavy chain and on CD151 recognition, they can easily be replaced by their human counterpart.

Considering the J-region, the human IGHJ6*01 gene contains 3 divergent residues as compared to both m214B2 and mIGHJ4*01. Since this region corresponds essentially to FR4, it can be fully humanized in a first instance (Fig. 9).

The Diversity D-gene corresponding essentially to the sequence of CDR3 (ARARSFYYAMDC) will be grafted without modification.

All the above described amino acids are important positions to consider. All combinations of human versus mouse residue for each of these positions will be considered during the humanization process. Selection of humanized forms will be based on their degree of humanness and conserved functional in vitro and in vivo properties.

### Example 5: Antibody specificity by western blot

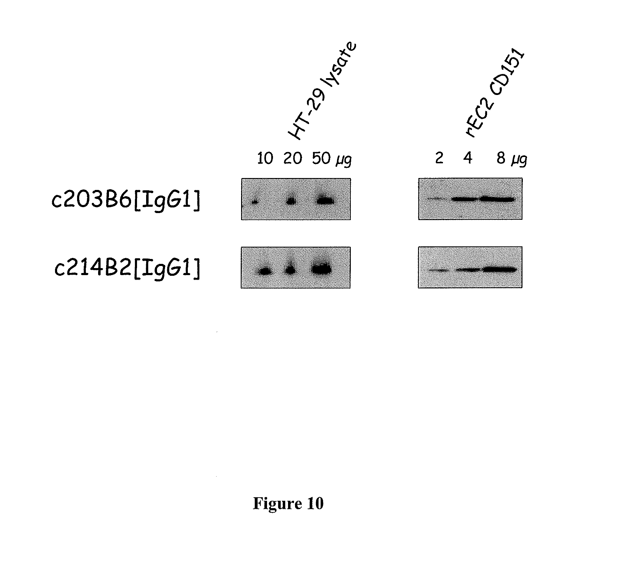

The specificity of the chimeric antibodies c214B2[IgG1] and c203B6[IgG1] was evaluated first by western blot. Briefly, purified recombinant large extracellular loop EC2 (2-8 µg) and HT-29 cell lysate (10-50 µg) were loaded on 12% acrylamide gels (BioRad). After electrophoresis under non reducing conditions, proteins were transferred to a nitrocellulose membrane, which was further incubated with the purified chimeric antibodies c214B2[IgG1] and c203B6[IgG1] at 0.5 µg/ml, and then with a peroxidase-conjugated rabbit polyclonal anti-human Ig (GE Healthcare). Proteins were detected by chemiluminescence. Both c214B2[IgG1] and c203B6[IgG1] were able to recognise full length CD151 by western blot (Figure 10). Moreover, they were also shown to be specific for the large extracellular loop, as assessed by their reactivity for the recombinant EC2 loop (Figure 10).

### Example 6: Competition experiments by ELISA

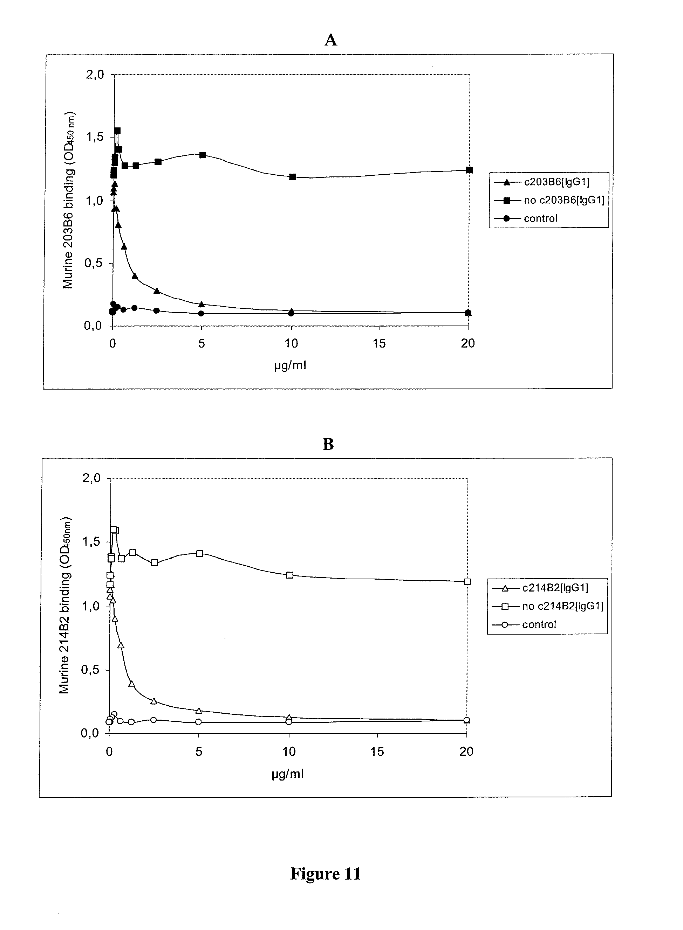

Cross-competition experiments were further performed by ELISA to evaluate the ability of the chimeric antibodies c214B2[IgG1] and c203B6[IgG1] to inhibit the binding of their corresponding murine forms to the recombinant EC2 loop. Briefly, 96-well ELISA plates were coated with recombinant EC2 at 5 µg/ml in PBS overnight at 4°C. Murine Mabs 203B6 and 214B2 at 80 ng/ml were further incubated in the absence or in the presence of increasing concentrations of their corresponding chimeric antibody forms, ranging from 0.01 to 20 µg/ml, for 1h at 37°C. For control experiments, no murine antibody was added. Horseradish peroxidase-conjugated polyclonal goat anti-mouse IgG was added at a 1/5000 dilution in PBS and incubated for 1 h at 37°C. After incubation with the peroxidase substrate TMB for 10 min at room temperature, the reaction was stopped with 1 M sulfuric acid and the optical density at 450 nm was measured. Figures 11A-11B show that the chimeric antibodies c214B2[IgG1] and c203B6[IgG1] were able to displace their murine forms of 214B2 and 203B6, respectively. IC₅₀ values, calculated by using the GraphPad Prism software, were 0.69 µg/ml and 0.71 µg/ml for c214B2[IgG1] and c203B6[IgG1], respectively.

### Example 7: Binding of chimeric antibodies to prostate cancer cells

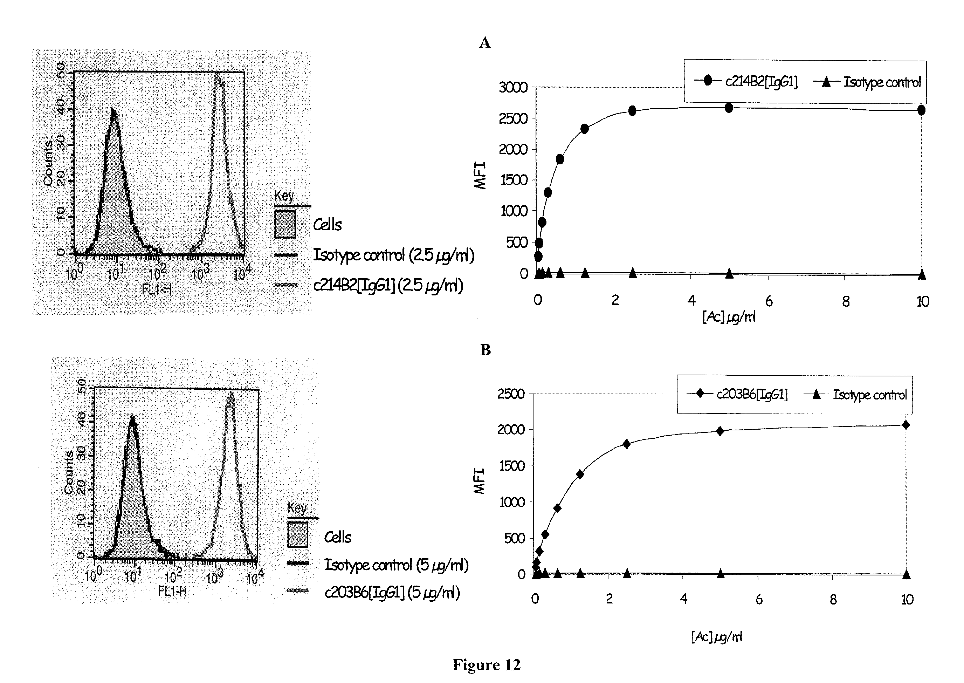

The binding of the chimeric antibodies c203B6[IgG1] and c214B2[IgG1] to the prostate cancer cells PC3 was assessed by flow cytometry. Briefly, PC3 cells were incubated at 1.10⁵ cells/100 µl in PBS buffer containing 1% BSA and 0.01% sodium azide, in the presence of varying concentrations of c203B6[IgG1] or c214B2[IgG1] for 20 min at 4°C. Cells were further washed and incubated with an Alexa488-conjugated goat anti-human antibody (Molecular Probes, 1/500 dilution in PBS) for 20 min at 4°C. Labelled cells were washed, centrifuged and resuspended in the previous buffer (150 µl) before analysis with a Facscalibur cytometer (Becton Dickinson). Propidium iodide was added to perform analyses on viable cells only. Binding of chimeric antibodies c203B6[IgG1] and c214B2[IgG1] to CD151 expressed at the surface of PC3 cells increased as a function of the antibody concentration (Figures 12A-12B). A plateau was reached at 2.5 µg/ml and 5 µg/ml for c214B2[IgG1] and c203B6[IgG1], respectively, indicating that the binding of these antibodies to PC3 cells is highly specific.

### Example 8: Antitumoral activity of a chimeric antibody c214B2]IgGl] against CD151 on the prostate PC3 xenograft model

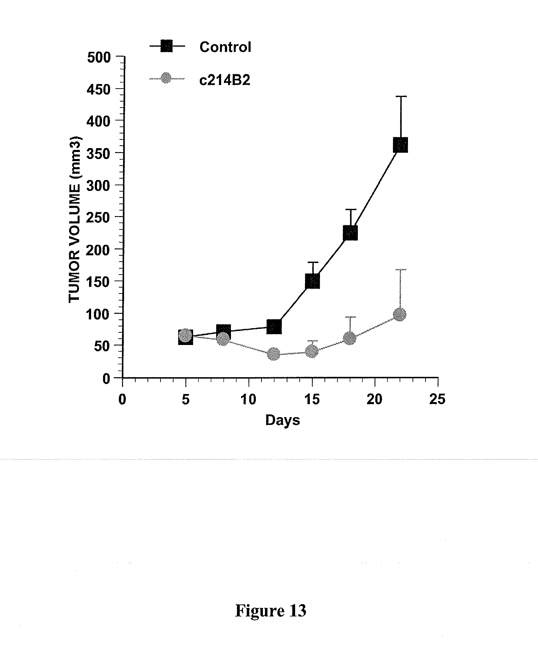

An over-expression of CD151 has been previously shown on prostate tissue using a tissue array analysis. To determine whether prostate cancer cells would be responsive to a targeted-CD151 therapy, the chimeric c214B2[IgG1], directed against CD151, has been tested, *in vivo,* in the PC3 xenograft model. The PC3 cell line is an androgen-independent cell line provided by the ATCC and grown in F12K medium supplemented with 10% FCS. Five million PC3 cells were implanted s.c. to 6 week old male Swiss mice. Five days after implantation, tumors were measurable and mice were randomized into 2 groups of 6 mice before starting i.p. injections with the c214B2[IgG1] chimeric antibody. A 2 mg/dose of c214B2 was injected as a loading dose and then twice a week injections at 1 mg/dose were performed. Tumour volume was evaluated twice a week and calculated with the following formula: π/6 X length X width X height. Results presented in figure 13 demonstrated that c214B2[IgG1], referred in this figure as c214B2, inhibited significantly *in vivo* tumor growth of PC3 cells. This result shows that targeting CD 151 could be an efficient therapy for cancer.

## Claims

1. Chimeric antibody, or a derived compound or functional fragment, **characterized in that** it comprises a light chain having a sequence comprising the amino acid sequence SEQ ID No. 7 or 25 and/or a heavy chain having a sequence comprising the amino acid sequence SEQ ID No. 8, 9, 26 or 27.

2. Chimeric antibody according to claim 1, **characterized in that** it comprises a light chain having a sequence comprising the amino acid sequence SEQ ID No. 7 and a heavy chain having a sequence comprising the amino acid sequence SEQ ID No. 8.

3. Chimeric antibody according to claim 2, **characterized in that** it is an IgG1.

4. Chimeric antibody according to claim 1, **characterized in that** it comprises a light chain having a sequence comprising the amino acid sequence SEQ ID No. 7, and a heavy chain having a sequence comprising the amino acid sequence SEQ ID No. 9.

5. Chimeric antibody according to claim 4, **characterized in that** it is an IgG4.

6. Chimeric antibody according to claim 1, **characterized in that** it comprises a light chain having a sequence comprising the amino acid sequence SEQ ID No. 25 and a heavy chain having a sequence comprising the amino acid sequence SEQ ID No. 26.

7. Chimeric antibody according to claim 6, **characterized in that** it is an IgG1.

8. Chimeric antibody according to claim 1, **characterized in that** it comprises a light chain having a sequence comprising the amino acid sequence SEQ ID No. 25 and a heavy chain having a sequence comprising the amino acid sequence SEQ ID No. 27.

9. Chimeric antibody according to claim 8, **characterized in that** it is an IgG4.

10. Isolated nucleic acid, **characterised in that** it is selected from the following nucleic acids:
a) a DNA or RNA nucleic acid coding for an antibody, or one of its derivative compounds or functional fragments, according to one of claims 1 to 9;
b) a nucleic acid complementary to a nucleic acid as defined under a);
c) a nucleic acid of at least 18 nucleotides capable of hybridising under conditions of high stringency with at least one of the nucleic acid sequences SEQ ID Nos. 16-18 or 34-36.

11. Vector comprising a nucleic acid according to claim 10.

12. Cell host comprising a vector according to claim 11.

13. Transgenic animal, with the exception of a human being, comprising a cell according to claim 12.

14. Composition comprising, as active ingredient, a compound consisting of an antibody, or one of its derived compound or functional fragments, according to one of claims 1 to 9.

15. Composition according to claim 14, **characterised in that** it additionally comprises, as a combination product for simultaneous, separate or time-staggered use, an antibody, a cytotoxic/cytostatic agent, a cell toxin or a radioelement.

16. Composition according to one of claims 14 or 15, as a medicament.

17. Use of an antibody, or one of its derived compound or functional fragments, according to one of claims 1 to 9 and/or of a composition according to any one of claims 14 to 16, in the preparation of a medicament intended for the prevention or treatment of cancer.

18. Use according to claim 17, **characterized in that** said cancer is a cancer selected from prostate cancer, lung cancer, colon cancer, breast cancer or pancreatic cancer.
